# EUROPÄISCHE PATENTSCHRIFT

(11) **EP 1 402 924 B1**
(45) Veröffentlichungstag und Bekanntmachung des Hinweises auf die Patenterteilung: **31.12.2008**
(21) Anmeldenummer: 02090315.9
(22) Anmeldetag: 04.09.2002
(51) Int. Cl.: A61P 9/00, A61K 31/56, A61K 31/568, A61K 31/58

(54) **Verwendung eines Mittel zur Therapie der Herzhypertrophie**
Use of a compound for the treatment of heart hypertrophy
Utilisation d'un composé pour le traitement de l'hypertrophie cardiaque

(43) Veröffentlichungstag der Anmeldung: 31.03.2004
(73) Patentinhaber: Fraunhofer-Gesellschaft zur Förderung der angewandten Forschung e.V., 80686 München (DE)
(72) Erfinder: Borlak, Jürgen, 31275 Lehrte/ OT Immensen (DE); Thum, Thomas, 30177 Hannover (DE)
(74) Vertreter: Baumbach, Friedrich

(56) Entgegenhaltungen:
- SHAH P M: "NEWER CONCEPTS IN HYPERTROPHIC OBSTRUCTIVE CARDIOMYOPATHY II" JAMA THE JOURNAL OF THE AMERICAN MEDICAL ASSOCIATION, CHICAGO,IL, US, Bd. 242, Nr. 16, 15. Oktober 1979 (1979-10-15), Seiten 1771-1776, XP009004532 ISSN: 0098-7484
- OI SHINJI ET AL: "Lovastatin prevents angiotensin II-induced cardiac hypertrophy in cultured neonatal rat heart cells" EUROPEAN JOURNAL OF PHARMACOLOGY, Bd. 376, Nr. 1-2, 2. Juli 1999 (1999-07-02), Seiten 139-148, XP002289966 ISSN: 0014-2999
- TOSIIIMA H ET AL: "COMPARABLE EFFECTS OF ORAL DILTIAZEM AND VERAPAMIL IN THE TREATMENTOF HYPERTROPHIC CARDIOMYOPATHY DOUBLE-BLIND CROSSOVER STUDY" JAPANESE HEART JOURNAL, JAPANESE HEART JOURNAL ASSOCIATION, JP, Bd. 27, Nr. 5, 1986, Seiten 701-715, XP001024687 ISSN: 0021-4868
- DATABASE BIOSIS [Online] BIOSCIENCES INFORMATION SERVICE, PHILADELPHIA, PA, US; 1994, STEWART J T ET AL: "Management of arrhythmias in hypertrophic cardiomyopathy" XP002289967 Database accession no. PREV199497362985 & CARDIOVASCULAR DRUGS AND THERAPY, Bd. 8, Nr. 1, 1994, Seiten 95-99, ISSN: 0920-3206

## Beschreibung

Die Erfindung betrifft die Verwendung eines Mittels für die Herstellung eines Arzneimittels zur Therapie der Herzhypertrophie. Anwendungsgebiete sind die Medizin und die pharmazeutische Industrie.

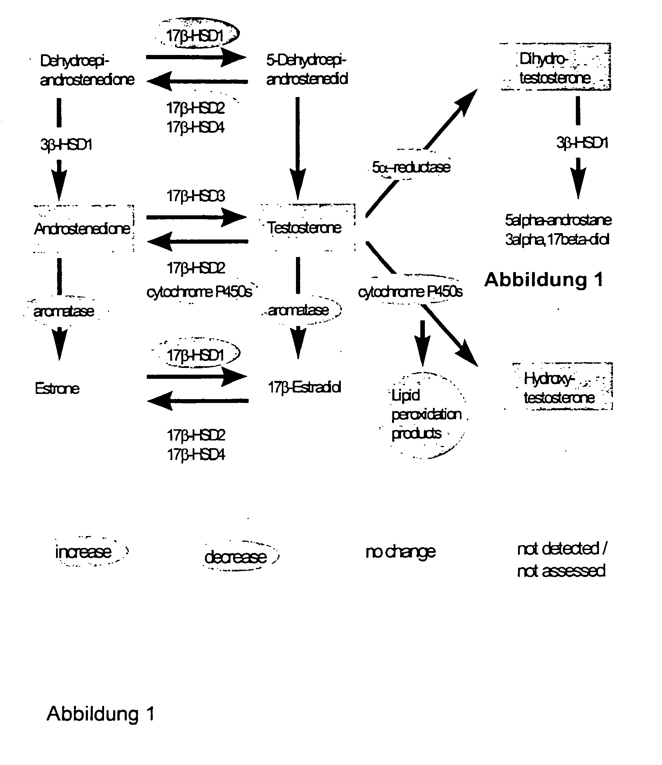

Testosteron ist ein Androgen und wird primär in den Leydig-Zellen des Hodens produziert. Testosteron kann direkt oder nach Modifikation durch Enzyme, wie beispielsweise durch Reduktion in Position 5 des Steroidgerüstes wirken. Das androgene Signaling (=Hormonwirkung) führt zur Ausbildung des männlichen Geschlechtstyps und nimmt Einfluß auf Stoffwechselprozesse der Talgdrüsen und akzessorischen Geschlechtsdrüsen (Prostata, Samenbläschen, etc.). Diese Effekte werden primär über 5-alpha-Dihydrotestosteron (5-alpha-DHT) vermittelt (Eicheler et al., 1998; Rosenfield et al., 1998; Schroder, 1994). Testosteron, sowie verschiedene Abkömmlinge regulieren die Spermiogenese, rekrutieren die für die Spermiogenese erforderlichen Substanzen, u.a. Fruktose, steigern den Eiweißaufbau, fördern das Knochenwachstum und erhöhen die Libido (O'Donnell et al., 1999; Cai et al., 1994; Arver et al., 1997; Ly und Handelsman, 2002; Mason und Morris, 1997). In der Prostata kann 5-alpha-DHT eine Hypertrophie auslösen (Schroder, 1994), weshalb durch 5-alpha-Steroid-Reduktase Hemmer eine zumindest partielle Rückbildung des hyperplastischen Drüsengewebes erzielt wird (Schulman, 2001). Derzeit werden 5-alpha-Steroid-Reduktase-Inhibitoren in der hormonellen Alopezie, Prostatahyperplasie und bei Prostatakarzinomen eingesetzt (Whiting, 2001; Schulman, 2001; Brawley et al., 2001).
Testosteron wird durch verschiedene Enzymsysteme im Körper verstoffwechselt, unter anderem durch das Cytochrom P450 Monooxygenase System, verschiedene 17-beta-Hydroxy-Steroid-Dehydrogenasen, die 3-beta-Hydroxy-Steroid-Dehydrogenase, Aromatase und die 5-alpha-Steroid-Reduktase (Abbildung 1). So wird Testosteron in den meisten Zielorganen zu 5-alpha-DHT reduziert, einem biologisch hochwirksamen Androgen und durch zytoplasmatische Rezeptoren gebunden in den Zellkern befördert, wo es zur Bildung eines Hormon-Rezeptor-Chromatin-Komplexes kommt (Porto et al., 1995). Infolge davon wird die Aktivierung spezifischer Gene eingeleitet, die zur mRNA Synthese und deren ribosomaler Übersetzung führen, was letztendlich eine de novo Proteinsynthese einleitet.

Sowohl die Spermatogenese als auch die Androgensekretion wird durch Hormone des Hypophysenvorderlappens gesteuert; Follikel-stimulierendes-Hormon (FSH) fördert die Spermatogenese durch Aktivierung der Sertoli-Zellen im Zusammenwirken mit Androgenen. Das Luteinisierende Hormon (LH) wirkt auf die Leydigschen Zellen und stimuliert hier die Androgenausschüttung. Die unter LH sezernierten Androgene wirken hemmend, sowohl auf die Ausschüttung von FSH, als auch auf die von LH. Ihre relativ schwache Hemmwirkung wird durch das im Hoden gebildete und im peripheren Gewebe aus Testosteron entstandene Östradiol unterstützt. Testosteroninjektionen in den Hypothalamus führen zu einer Hodenatrophie und einer verminderten Ausschüttung eines gemeinsamen Releasing-Hormons für FSH und LH. Inhibitoren der Androgenausschüttung sind vor allem Östrogene und in schwächerem Maße auch Gestagene. Sie hemmen die Ausschüttung gonadotroper Hormone über einen feed-back Mechanismus. So führt das Fehlen von FSH zum Sistieren der Spermatogenese, während ein Mangel an LH die Androgensekretion zum Erliegen bringt (Sairam und Krishnamurthy, 2001). Die Verabreichung von Östrogenen beim Mann bewirkt also eine Art "hormonelle Kastration".

Um die Wirkungen der Androgene verhindern zu können, werden unter anderem chemisch synthetisierte Antiandrogene als Rezeptorblocker eingesetzt. Diese können in Folge ihrer Testosteron-ähnlichen Struktur an den Androgenrezeptor binden und dabei die Androgenrezeptor vermittelte Signaltransduktion verhindern. Bekannte Beispiele sind das Cyproteron und das Cyproteronacetat (Poujol et al., 2000). Unter der Einwirkung von Antiandrogenen kommt es nicht zur Ausbildung des Samenleiters und seiner Anhangsdrüsen, sowie des Penisses (Krause, 1976). Auch die Differenzierung des Sexualzentrums im Hypothalamus zum männlichen Phänotyp bleibt aus. Bei adulten Tieren wirken Antiandrogene hemmend auf die Spermatogenese und Samenblase, Prostata und Talgdrüsen atrophieren unter der Einwirkung von Antiandrogenen (Miyata et al., 2002). Darüber hinaus führen Antiandrogene zu einer gesteigerten Sekretion des FSH- LH- Releasing-Hormons und demzufolge zu einer vermehrten Ausschüttung von FSH und LH.

Eine endokrine Überfunktion der Hoden durch Adenome der Leydigschen Zellen kommt nur selten vor und führt in jugendlichem Alter zur Pubertas praecox, einer vorzeitig einsetzenden Pubertät.

Therapeutisch werden Androgene allgemein bei Androgen-Mangel, z.B. nach Kastration, beim Klimakterium virile, bei Impotentia generandi infolge von Oligospermie und bei der Impotentia coeundi verabreicht. Die Einnahme von Anabolika (z.B. Testosteron und Dihydrotestosteron) führt zu verstärktem Muskelaufbau (Sinha-Hikim et al., 2002). Die molekularen Ursachen sind aber größtenteils unbekannt.

Gegenwärtig wird in multizentrischen klinischen Studien der therapeutische Nutzen einer Testosteron-Substitution in der "männlichen Menopause" untersucht. Interims-Analysen belegen einen verstärkten Knochen- und Muskelaufbau und eine Verringerung des Körperfettgehaltes nach Testosteron-Supplementierung (Howell und Shalet, 2001). Die Verabreichung von 5-alpha-DHT führte dagegen zu einem Absinken der Testosteron-Plasmaspiegel, sowie der LH- und FSH Spiegel (Ly et al., 2001).

Die Biosynthese von Testosteron und Dihydrotestosteron erfolgt ähnlich wie die der Kortikosteroide aus dem Cholesterinstoffwechsel über delta-5-Pregnenolon. Beim Menschen verläuft der weitere Syntheseweg vor allem über die Hydroxylierung (Hydroxypregnenolon) und weiteren Verstoffwechselung zu Dihydroepiandrosteron. Testosteron wird in den meisten Zielorganen zu Dihydrotestosteron reduziert und dann über komplexe Oxidationsprodukte stereo- und regioselektiv oxidiert bzw. hydroxiliert. Untersuchungen von Thum und Borlak (2000) an isolierten Herzmuskelzellen der Ratte, sowie subzellulären Fraktionen (Mikrosomen) des menschlichen Herzens haben dokumentiert, dass Testosteron zu verschiedenen Metaboliten (Oxidation, Reduktion, Isomerisation, etc.) verstoffwechselt wird. Einige der metabolischen Abbauprodukte, wie beispielsweise das Dihydrotestosteron besitzen die Fähigkeit zur Bindung an den Androgenrezeptor. Nach Liganden-Rezeptor-Interaktionen wird der Androgenrezeptor in den Zellkern transloziert, und nach entsprechenden Modifikationen kommt es zur nicht kovalenten Bindung an responsive Elemente zahlreicher Targetgene (Porto et al., 1995). Das Wechselspiel zwischen verschiedenen Transkriptionsfaktoren [u.a. Hierarchie und Netzwerk, sowie Protein/Protein Interaktionen] und des Androgenrezeptors in der transkriptionellen Aktivierung von Androgenrezepotor-responsiven Genen ist aber größtenteils unbekannt. So gibt es Hinweise, dass eine transiente Therapie von preterm und neugeborenen Kindern mit dem Glukokortikoid Dexamethason zu einer Herzhypertrophie führt (Skelton et al., 1998). Des weiteren ist bekannt, dass Frauen vor der Menopause (praemenopausal) ein geringeres Risiko für Herzkreislauferkrankungen haben. Möglicherweise wirkt Östrogen kardioprotektiv (Rosano und Panina, 1999). Dieser Schutz nimmt postmenopausal mit fallendem Östrogenspiegel ab. In der weltweit größten Studie (Women Health Study) mit über 16000 postmenopausalen Frauen wurde der therapeutische Nutzen einer kombinierten Hormonsubstitutionstherapie (Östrogen und Progesteron) untersucht. Erstaunlicherweise führte die Östrogen und Progesteron-Kombinationstherapie zu einem >20% erhöhtem Risiko für Herzkreislauferkrankungen, sowie Schlaganfällen und invasivem Brustkrebs und es wurde ein etwa 50% erhöhtes Thromboserisiko beobachtet wurde (JAMA, 2002, 288, 321-333).

Wie zuvor beschrieben, wird Testosteron in zahlreichen Organen verstoffwechselt und ist für die Ausprägung des männlichen Phänotyps von entscheidender Bedeutung. Metabolische Abbauprodukte des Testosterons sind unter anderem das Dihydrotestosteron, welches effektiv an den Androgenrezepor bindet und damit eine Aktivierung des Androgenrezeptors bewirkt. Darüber hinaus ist bekannt, dass der Androgenrezeptor bestimmte Gene in der transkriptionellen Regulation steuert und damit unmittelbar Einfluß auf deren transkriptionellen Aktivierung nimmt. Weiterhin wurde bereits erwähnt, dass Cytochrom P450 abhängige Monooxygenasen wesentliche Funktionen im Steroidstoffwechsel wahrnehmen und für die regio- und stereoselektive Hydroxylierung verantwortlich sind (Arlotto et al., 1991). Dabei nimmt der gewebsspezifische Metabolismus durch Cytochrom P450 Monooxygenasen eine Sonderfunktion ein, zumal die verschiedenen Isoformen nicht ubiquitär exprimiert werden und deshalb in den verschiedenen Geweben und Organen mit unterschiedlichen Stoffwechselleistungen und unterschiedlichen metabolischen Abbauprodukten zu rechnen ist. Die Wirkungen dieser Stoffwechselprodukte hängt deshalb von den einzelnen Zelltypen und Geweben ab, wobei dem Androgenrezeptor eine besondere Bedeutung zukommt. Demzufolge ist das Vorhandensein und die Rezeptordichte des Androgenrezeptors von entscheidender Bedeutung für die Androgen- und Androgenrezeptor-Liganden vermittelte biologische Signalübertragung.

Die Entstehung und Progression der kardialen Hypertrophie wird als multifaktoriell angesehen und es werden derzeit verschiedene Hypothesen diskutiert. Zum einen führt eine überhöhte Dehnung der Herzmuskelzellen zur Aktivierung verschiedener Rezeptoren, wie beispielsweise G-gekoppelte oder Tyrosin-Kinase abhängige Rezeptoren, die dann zu einer verstärkten Synthese Herzmuskelschädigender Proteine führen (Sugden und Bogoyevitch, 1995). Des weiteren kommt es zur verstärkten Produktion von Wachstumsfaktoren, wie beispielsweise die übersteigerte Sekretion von fibroblastic growth factor oder transforming growth factor beta, die dann wiederum zu einer zellulären Hypertrophie der Herzmuskelzellen führen (Parker et al., 1990; Roberts et al., 1992). Inwieweit Hormone bei der Entstehung der Herzhypertrophie eine Rolle spielen, ist jedoch bislang unbekannt.
In English et al. Circulation. 2000 102 1906-11 ist zwar bereits beschrieben, dass die transdermale Applikation von Testosteron zu einer koronaren Vasodilatation (Erweiterung der Herzkranzgefäße) führt, und somit das Herz besser durchblutet wird. Jedoch machen die Autoren keinerlei Angaben über mögliche Mechanismen und Effekte auf das Herzgewebe, z.B. Fehlwachstum bei einer Herzhypertrophie. Diese Publikation erwähnt keine gewebespezifische kardiale Testosteronverarmung im Rahmen von Herzerkrankungen allgemein, und bei Herzhypertrophie im speziellen. Es sind lediglich die Effekte von Testosteron auf die Herzkranzgefäße, nicht aber auf die Herzmuskulatur beschrieben.
Aus der Literatur sind bereits Behandlungsansätze bekannt, die an eine Veränderung des Testosteronlevels ansetzen.
So beschreibt beispielsweise Shapiro et al. Am J Ther. 1999 6 167-74 die Verbesserung des Cholesterin-Metabolismus unter Testosteron-Therapie. Bei einer Testosteron-Therapie sinken der LDL- und Cholesterinspiegel im Plasma, wogegen das HDL-Level ansteigt. Daher ist anzunehmen, dass Testosteron einen anti-arteriosklerotischen Effekt bewirkt. In diesem Dokument wird an keiner Stelle erwähnt, welche Effekte Testosteron auf das Herz hat, schon gar nicht im Zusammenhang mit Herzhypertrophie.
Ebenfalls bekannt sind bereits einige Ansätze zur Therapie von Gefäß-und Herzerkrankungen, von denen jedoch noch keiner zu einer befriedigenden Wirkung bei der Behandlung, insbesondere der Herzhypertrophie, geführt hat.
So beschreibt Shah, J. American Medical Association 1979 242 1771-6 die Verwendung von β-Adrenorezeptor hemmenden Stoffen und insbesondere dem Betablocker Propranolol zur Behandlung von hypertropher obstruktiver Kardiomyopathie. Wie hier ausgeführt wird, haben sich Medikamente, die auf einem anderen Prinzip basieren, für die Langzeitanwendung bei dieser Krankheit als nicht hilfreich erwiesen. Wie aus dem Dokument hervorgeht, wird die zusätzliche Verwendung von Chinidin-Verbindungen ausschließlich bei der Indikation einer Arrhythmie empfohlen und nicht bei der Obstruktion selbst. Es werden keine Substanzen beschrieben, die gezielt die Konzentration von Testosteron in erkranktem Gewebe zur Behandlung einer Obstruktion beeinflussen und auf den Normalwert erhöhen.
Oi et al. Eur J Pharmacology 1999 376 139-48 beschreibt die Stimulation von Zellkulturen mit dem Oktapeptid Angiotensin II, das mit Angiotensin (AT)-Rezeptoren interagiert. Durch diese Interaktion wird in der Zelle eine Signalkaskade induziert, die in der Aktivierung von p21 ras mündet. P21 ras vermittelt daraufhin die Aktivierung von MAP-Kinasen, die schließlich Faktoren aktivieren, die Zellwachstum einleiten. Daher wird die Aktivität von MAP-Kinasen auch als Indikator für die von Angiotensin verursachte Hypertrophie verwendet. Ansatzpunkt zur Reduzierung der Angiotensin-Hypertrophie ist in diesem Dokument die durch Lovastatin vermittelte Inhibierung eines Co-Faktors (HMG-CoA), der bei der Aktivierung von p21 ras entscheidend ist. Es wird gezeigt, dass Lovastatin zwar deutlich die Aktivität von p21 ras in stimulierten Zellkulturen reduzieren kann, bei dem zur Beurteilung des Effekts auf die Hypertrophie wichtigen Marker "MAP-Kinase-Aktivität" ist jedoch kein Effekt (in Kombination mit Angiotensin II und Mevalonat) oder nur ein geringer Effekt (in Kombination mit Angiotensin II) gezeigt.
In der Publikation Toshima et al. Jpn Heart J. 1986 27 701-715 ist die Anwendung von Diltiazem als Calcium-Kanal-Blocker bei Patienten mit hypertropher Kardiomyopathie in zwei Perioden beschrieben. Die Ergebnisse fallen je nach betrachteter Periode gegensätzlich aus. Während in Sequenz A von sechs Patienten mit Symptome fünf eine subjektive Verbesserung angeben, nimmt die Anzahl der Patienten mit Symptomen in der zweiten Periode der Behandlung mit Diltiazem sogar noch zu. Auch in dieser Publikation wird keine Behandlung von auslösenden Faktoren der Herzhypertrophie sowie keine Erhöhung der zellulären Testosteronkonzentration offenbart.
In dem Dokument Stewart et al. Cardiovasc Drugs Ther. 1994 8 95-9 ist eine Behandlung von Symptomen der Kardiomyopathie vorgestellt, nämlich von Herzrhythmusstörungen und insbesondere von Herzvorhofflimmern. Wie in dem Dokument dargestellt wird, kann Amiodaron die Rückkehr des Herzflimmerns zu einem Sinus-Rhythmus fördern, auch wenn der diesem Prozess zugrunde liegende Mechanismus noch nicht systematisch bestätigt wurde. Dahingegen wird keine Erhöhung der Testosteronkonzentration und keine direkte Beeinflussung der Herzhypertrophie festgestellt, sondern lediglich die Anwendung von Amiodaron bei Herzrhythmusstörungen angeraten.

Der Erfindung lag die Aufgabe zugrunde, prinzipiell neue Möglichkeiten zur Therapie der Herzhypertrophie, aufzufinden. Ziel der Erfindung ist die Bereitstellung eines Mittels zur Herstellung eines Arzneimittels mit dem die Herzhypertrophie wirkungsvoll behandelt werden kann.

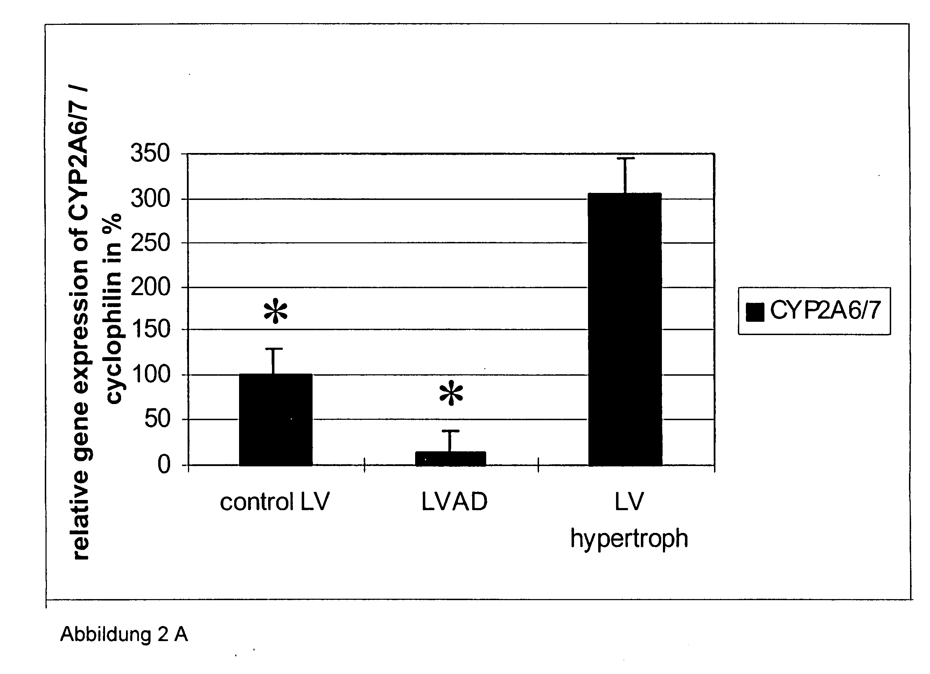

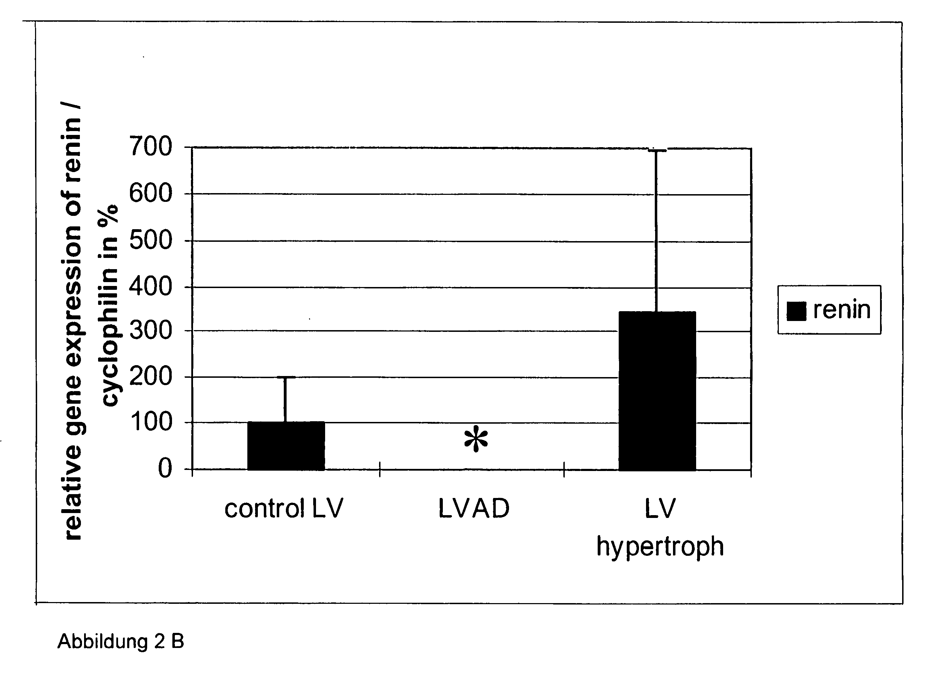

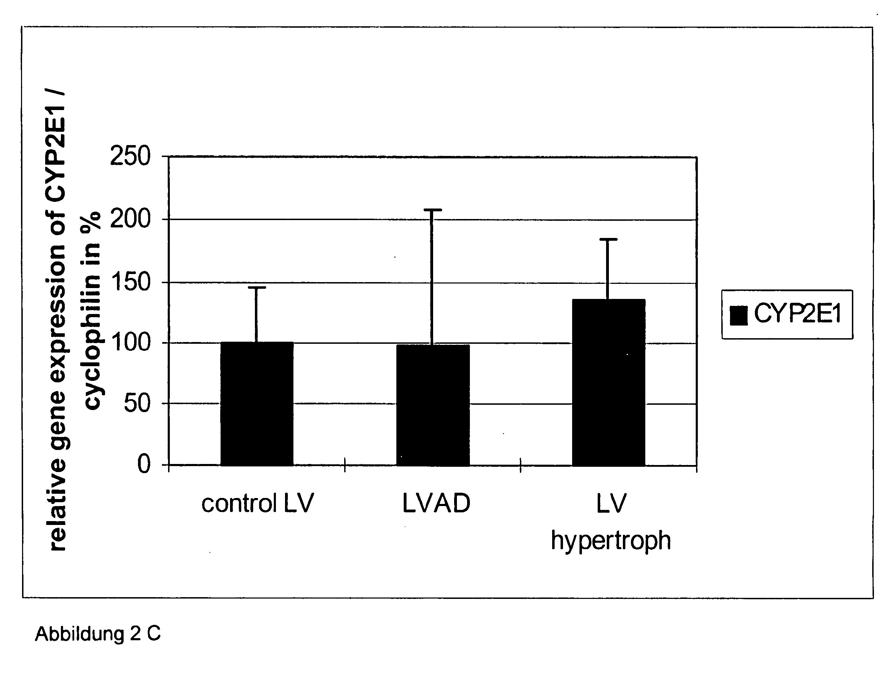

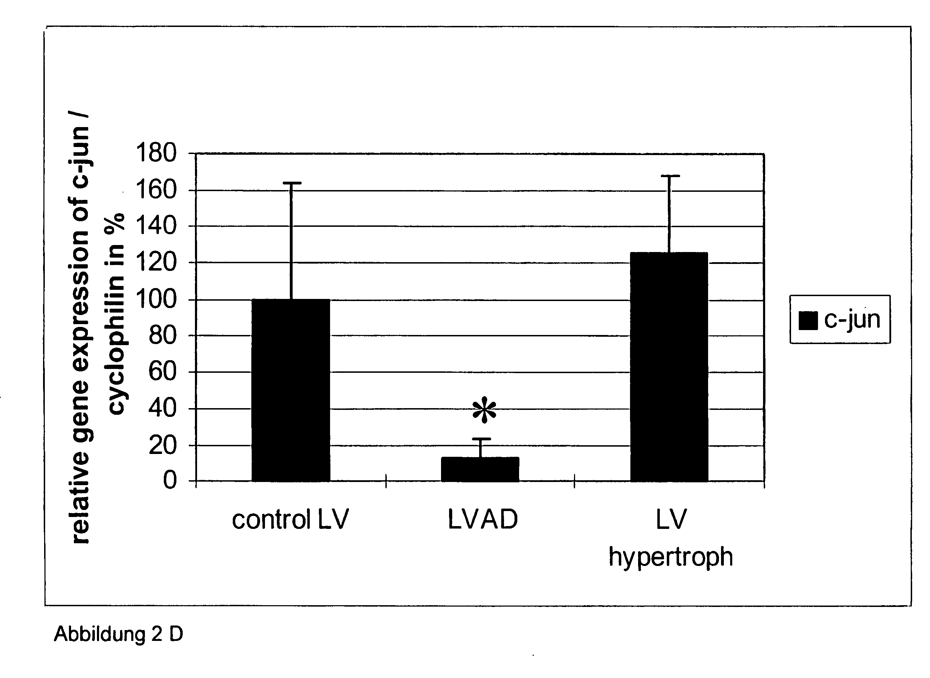

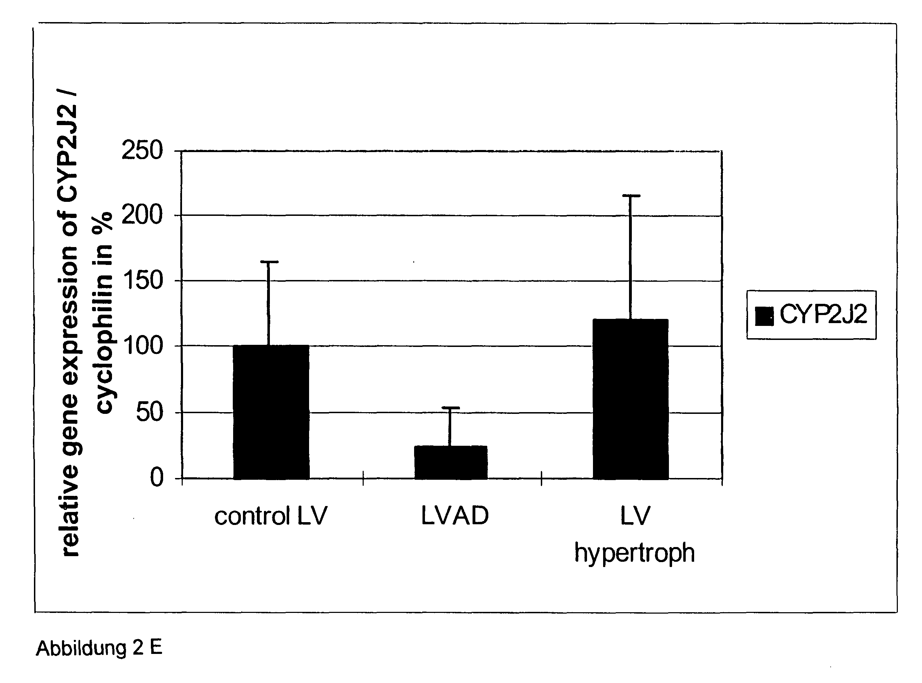

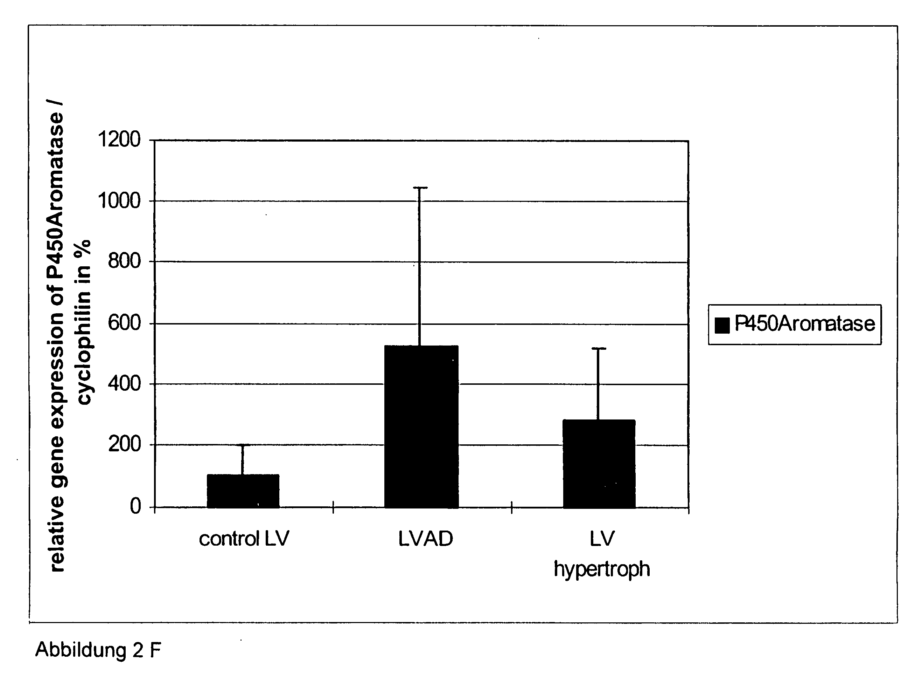

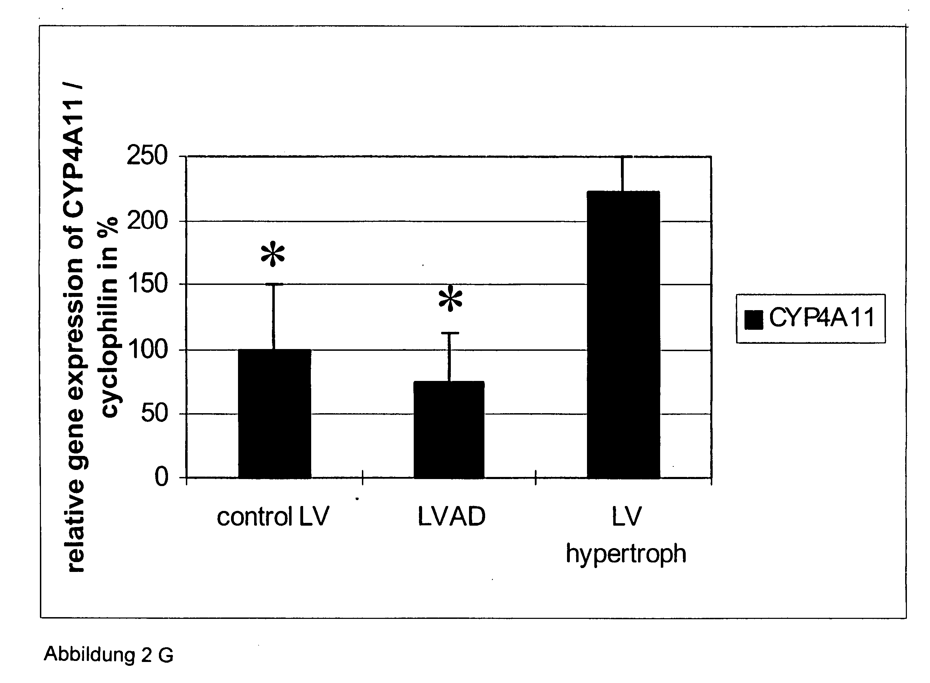

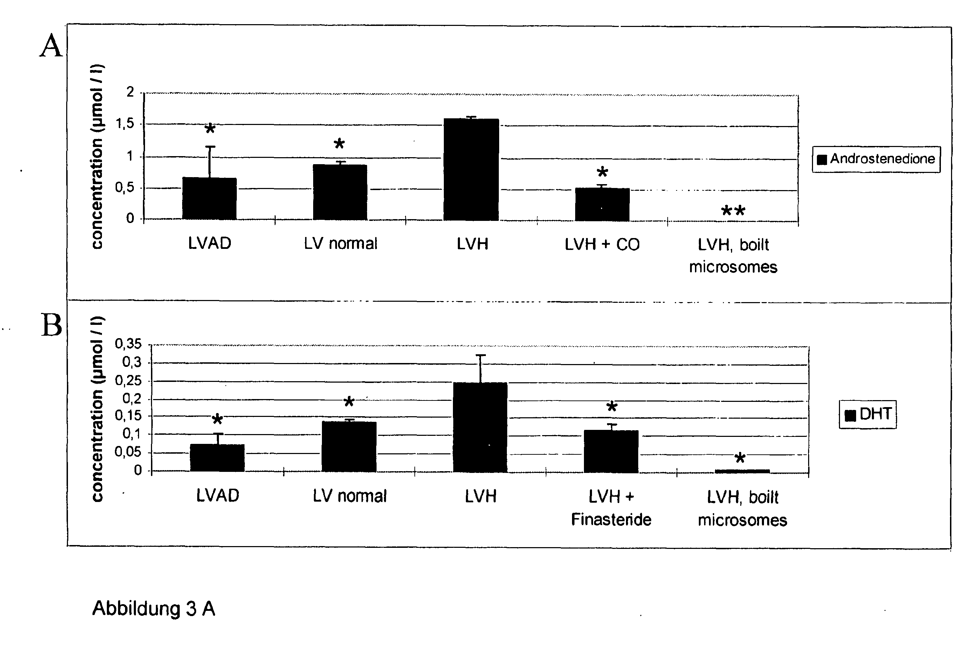

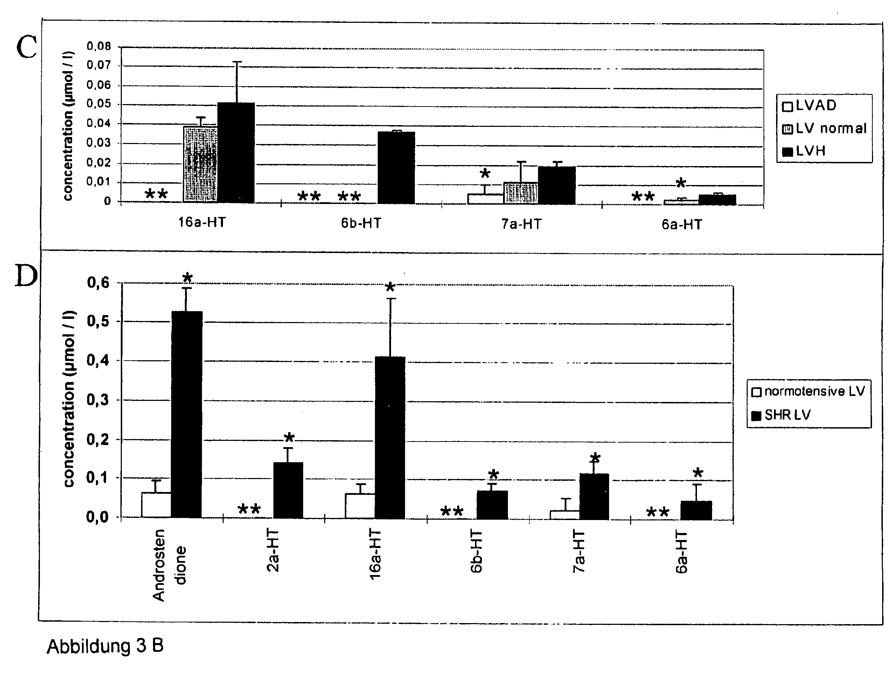

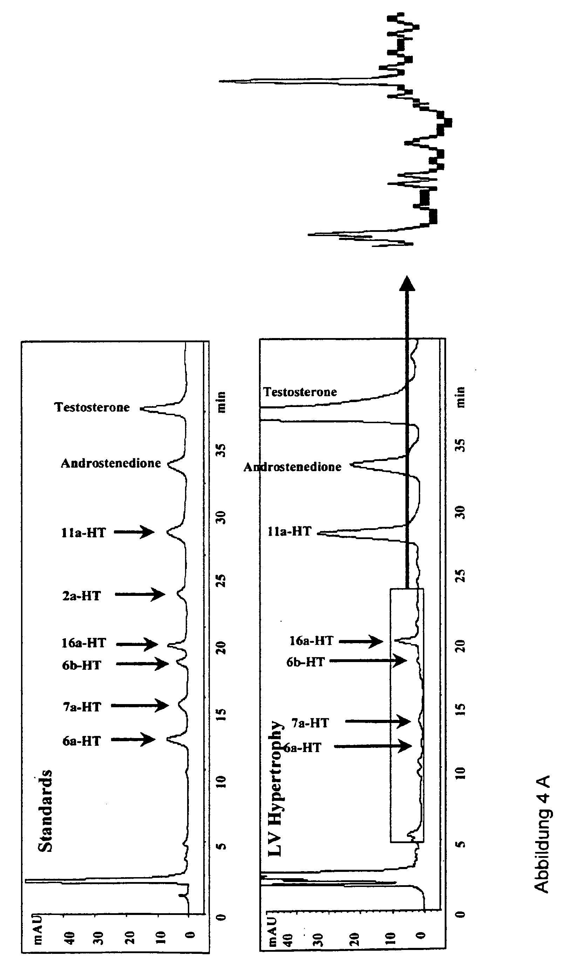

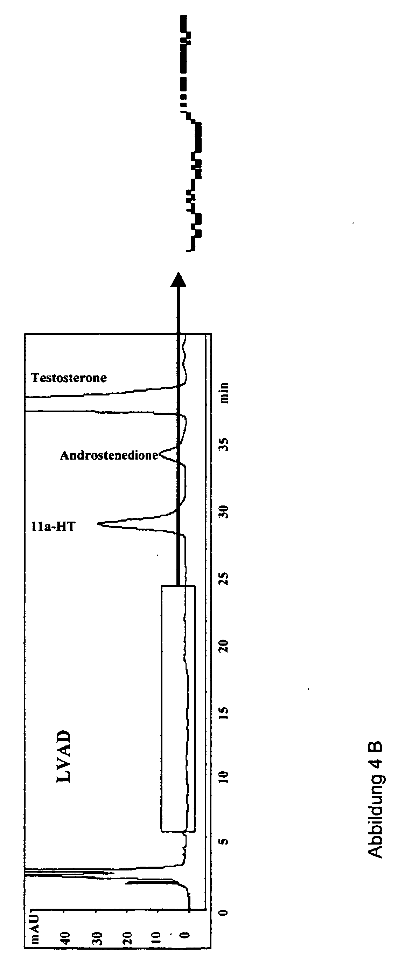

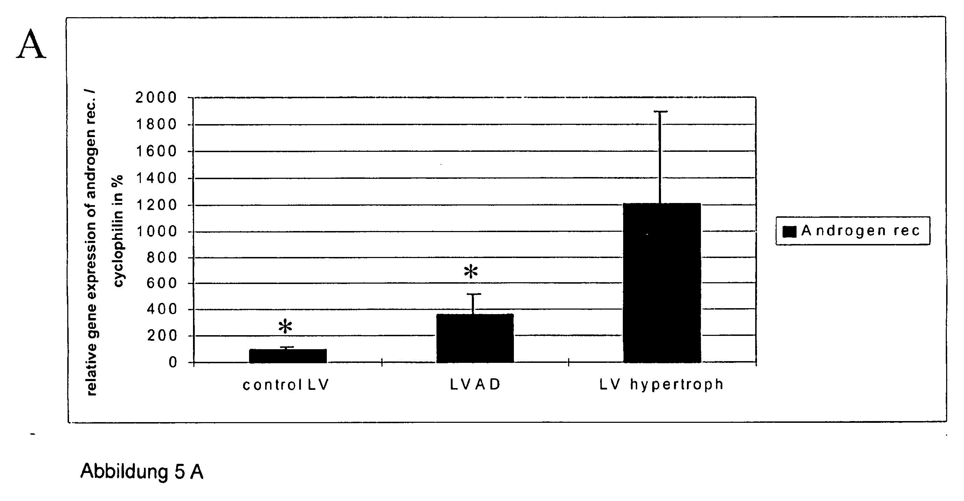

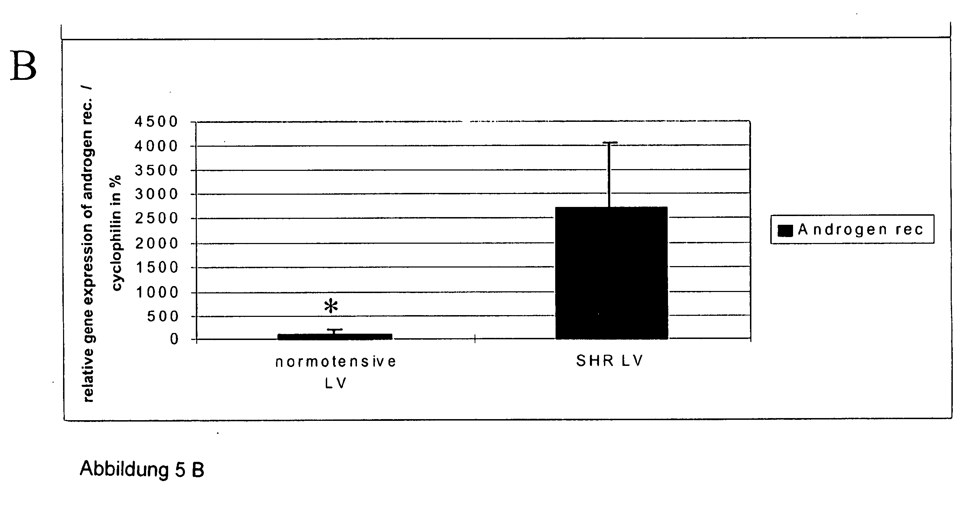

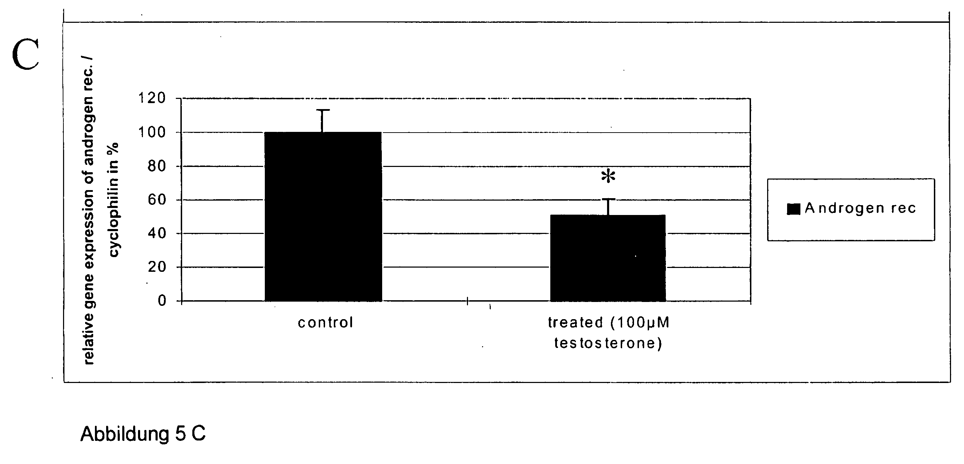

Die Erfindung basiert auf umfangreichen Gen- und Proteinexpressions-Studien. Es wurden vergleichende Untersuchungen an explantierten Herzen von Patienten mit differentialdiagnostisch etablierter Herzhypertrophie, und an gesunden Herzen, die nicht für die Transplantation freigegeben wurden, durchgeführt (8 Patienten mit dilatativer Kardiomyopathie (DCM), wovon n=5 ein linksventrikuläres Assist Device (LVAD) für über 6 Monate implantiert hatten und n=3 Patienten mit DCM und kombinierter Herzhypertrophie). Dabei wurden völlig unerwartete und überraschende Untersuchungsergebnisse erhalten, welche eine stark veränderte Verstoffwechselung des Testosterons im hypertrophen Herzgewebe belegen. Es wurde eine hochsignifikante Zunahme / Bildung von 5-alpha-Dihydrotestosteron, sowie von weiteren P450 katalysierten stereo- und regioselektiven Oxidations- und Hydroxilierungs-Produkten beobachtet. Im Vergleich normal versus hypertrophiertes Herzmuskelgewebe zeigen die Untersuchungen, dass Gene, die für verschiedene P450 Monooxygenase Isoformen kodieren, wie beispielsweise CYP2A6/7, CYP4A11, P450 Aromatase, aber auch Renin im hypertrophiertes Herzmuskelgewebe verstärkt exprimiert werden (Abbildung 2). Die hochsignifikante Induktion der P450 Monooxygenase-Expression führt zur verstärkten Anreicherung deren translatierter Proteine, die ursächlich für den veränderten Testosteronmetabolismus verantwortlich gemacht werden können. Die Untersuchungen belegen eine entgleiste / übersteigerte Produktion von 5-alpha-Dihydrotestosteron, Androstendion, sowie weiterer stereo- und regioselektiver Hydroxyderivate im hypertrophen humanen Herzgewebe, die in der biologischen Signalgebung der Krankheitsentstehung eine kausale Rolle einnehmen (entweder Rezeptorvermittelt oder davon unabhängig; Abbildungen 3 und 4). Eine Hemmung der 5-alpha-Reduktase mit Finasteride führt zu einer Reduktion und Normalisierung der Dihydrotestosteron-Bildung im Herzgewebe (Abbildung 3). Die verstärkte Verstoffwechselung des Testosterons führt zu einem starkem Absinken der Testosterongewebsspiegel und zu einer statistisch hochsignifikanten (>12-fach) Induktion der Androgenrezeptor-Expression (Abbildung 5). Die Induktion des Androgenrezeptors ist eine biologische Antwort auf die zunehmende Verarmung des Testosteronsgewebsspiegels im hypertrophen Herzen.

Weitere Experimente mit spontan hypertensiven Ratten (SHR), einem etablierten Krankheitsmodell für die Herzhypertrophie, bestätigen die bereits im hypertrophen humanen Herz ermittelten Befunde (Abbildungen 3, 5 und 6). Im Herzgewebe des SHR-Stamms ist der Testosteronstoffwechsel ebenfalls stark verändert, und es wurde ein hochsignifikanter Anstieg an stereo- und regioselektiven Metaboliten des Testosterons (Abbildung 3) beobachtet. Im Vergleich zu normotensiven Ratten wird im hypertrophen Herzgewebe der erkrankten Ratten eine nahezu 30-fache Induktion des Androgenrezeptors beobachtet (Abbildung 5). Auch hier führt die verstärkte Verstoffwechselung des Testosterons zu verringerten Testosterongewebsspiegeln im Herzmuskel, und die Gewebsarmut an Testosteron führt zur verstärkten Expression des Androgenrezeptors. Eine Testosterongewebsverarmung führt zu einer Induktion des Androgenrezeptors im Herzen.

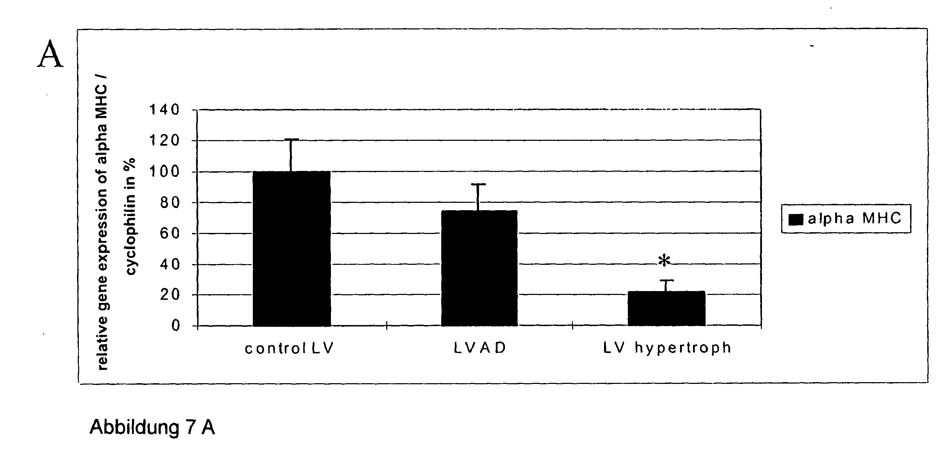

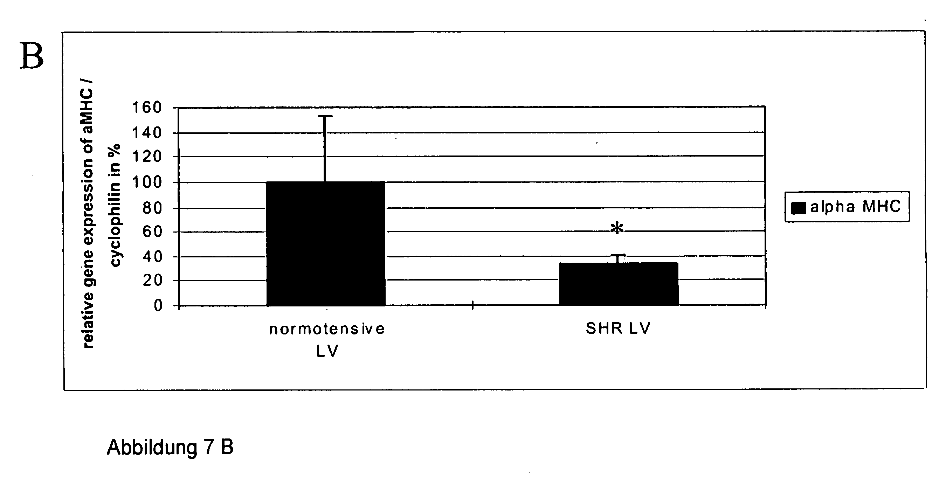

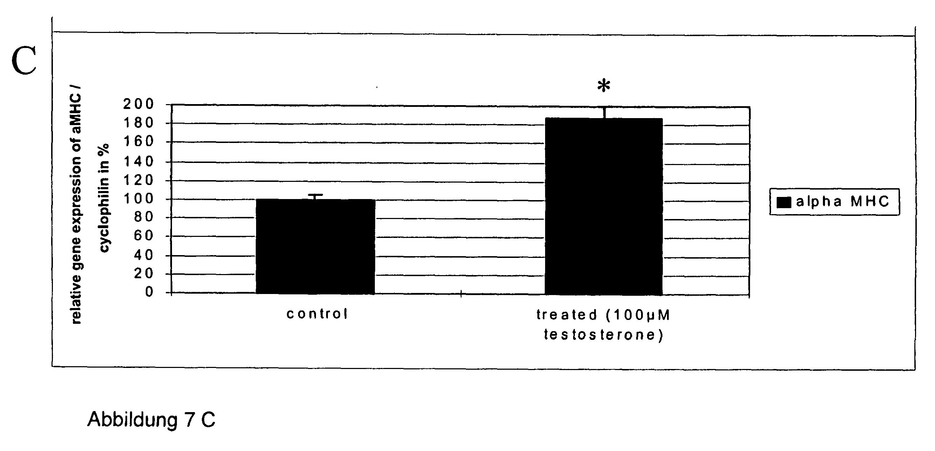

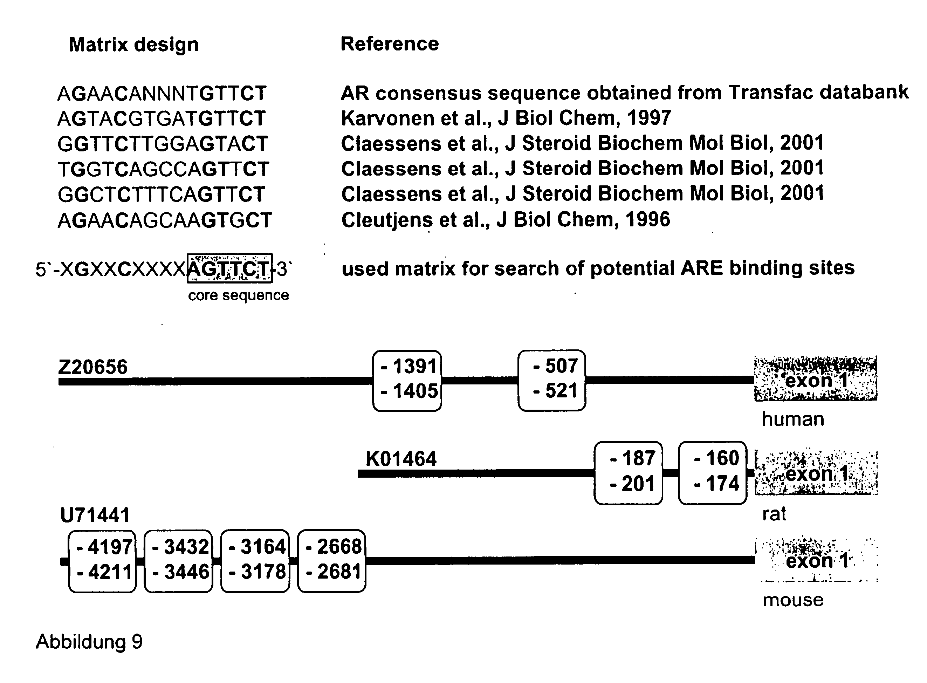

Weitere Untersuchungen belegen, dass die Expression des alpha-MHC im erkrankten / hypertrophierten Herzgewebe stark reduziert ist (Abbildung 7). Das alpha-MHC kodiert für ein wichtiges Strukturprotein im Herzmuskel und wird als diagnostischer Krankheitsmarker in der Differentialdiagnose der Herzhypertrophie bereits in akademischen Lehrkrankenhäusern eingesetzt (Nachweis in Biopsiematerial; Nakao et al., 1997). Bioinformatorische Analysen der Promotorregion des alpha-MHC kodierenden Gens belegen das Vorhandensein mehrerer Androgenrezeptor-Bindungsstellen (siehe Abbildung 9). Damit kann gezeigt werden, dass Testosteron selbst, aber auch Liganden, die zu einer Aktivierung des Androgenrezeptors führen, die alpha-MHC Genexpression steuern. Ein verminderter Testosterongewebsspiegel, wie er insbesondere in der Herzhypertrophie vorliegt, führt aber zu einer verminderten Expression des alpha-MHC.

Aufgrund dieser Untersuchungsergebnisse wird das erfindungsgemäße Mittel zur Herstellung eines Arzneimittels zur Therapie der Herzhypertrophie, zur Verfügung gestellt.

Die Erfindung wird durch das in den Ansprüchen beschriebene Mittel realisiert.

Das Mittel zur Herstellung eines Arzneimittels zur Therapie der Herzhypertrophie, ist dadurch gekennzeichnet, dass damit die Testosteron-Konzentration in den erkrankten Geweben auf den Normalwert erhöht wird.

Die Testosteron-Konzentration in den erkrankten Geweben kann durch Zugabe mindestens einer Substanz aus den folgenden Gruppen:
- Testosteron;
- Substanzen mit testosteronähnlichen Wirkungen;
- Testosteron-Mimetika;
- Substanzen, die die Testosteron-Synthese fördern;
- Substanzen, die die Verstoffwechselung des Testosterons hemmen, auf den Normalwert erhöht werden.

Die Folgeprodukte des Testosteronabbaus können durch Zugabe mindestens einer Substanz aus den folgenden Gruppen:
- Substanzen, die an den Androgenrezeptor binden und eine Regulierung im Sinne einer Normalisierung der Rezeptordichte
   bewirken;
- Substanzen, die an den Androgenrezeptor binden und eine Regulierung im Sinne einer Hemmung der Androgenrezeptor
   vermittelten Genexpression, wie sie beispielsweise in der Herzhypertrophie beobachtet wird, bewirken,
gehemmt und/oder eliminiert werden.

Das erfindungsgemäße Mittel zur Herstellung eines Arzneimittels zur Therapie der Herzhypertrophie, ist dadurch gekennzeichnet, dass es Substanzen enthalten, die die Testosteron-Konzentration in den erkrankten Geweben auf den Normalwert erhöhen bzw. Folgeprodukte des Testosteronabbaus hemmen und/oder eliminieren.

Das Mittel enthält mindestens eine Substanz aus den nachfolgend genannten Gruppen, die die Testosteron-Konzentration in den erkrankten Geweben auf den Normalwert erhöhen:
- Testosteron, vorzugsweise in Form von Testosteron-Estern, 19-Nortestosteron, Testosteronpropionat, Testosteronundecanoat, Mesterolon, Fluoxymesteron;
- Substanzen mit testosteronähnlicher Wirkungen vorzugsweise Nandrolondecanoat, Clostebolacetat, Metenololonacetat, Androstendion, Dehydroepiandrosteron;
- Testosteron-Mimetika;
- Substanzen, die die Testosteron-Synthese fördern, vorzugsweise synthetische Gonadoliberin-Analoga und weitere nichtsteroidale Substanzen, die eine verstärkte Testosteron Ausschüttung fördern, sowie Antagonisten der Gonadotropin Releasing Hormon inhibierenden Hormone;
- Substanzen, die die Verstoffwechselung des Testosterons, insbesondere die Bildung von Dihydrotestosteron, hemmen, vorzugsweise:
   a) Cytochrom P450 (CYP)-Monooxygenasen Hemmer; und / oder
   b) Steroid-Reduktasen Hemmer; und / oder
   c) Isomerasen Hemmer; und / oder
   d) 17-beta-Hydroxy-Steroid-Dehydrogenasen Hemmer; und / oder
   e) 3-beta-Hydroxy-Steroid-Dehydrogenasen Hemmer; und / oder
   f) Aromatase Hemmer.

Als Cytochrom P450 (CYP)-Monooxygenasen Hemmer werden unter anderem: Amiodaron, Cimetidin, Fluoroquinolone, Fluvoxamin, Furafyllin, Methoxsalen, Mibefradil, Ticlopidin, Thiotepa, Cimetidin, Felbamat, Fluoxetin, Indomethacin, Ketoconazol, Lansoprazol, Modafinil, Omeprazole, Paroxetin, Probenicid, Topiramat, Fluconazol, Fluvastatin, Isoniazid, Lovastatin, Phenylbutazon, Sertralin, Sulfamethoxazol, Sulfaphenazol, Teniposid, Trimethoprim, Zafirlukast, Clecoxib, Chlorpromazin, Chlorpheniramin, Clomipramin, Kocain, Doxorubicin, Halofantrin, Red-Haloperidol, Levomepromazin, Metoclopramid, Methadon, Mibefradil, Moclobemid, Quinidin, Ranitidin, Ritonavir, Terbinafin, Diethyl- Dithiocarbamat, Disulfiram, Delaviridin, Indinavir, Nelfinavir, Ritonavir, Saquinavir, Ciprofloxacin, Clarithromycin, Diltiazem, Erythromycin, Gestoden, Grapefruit Saft, Itraconazol, Mifepriston, Nefazodon, Norfloxacin, Norfluoxetin oder Troleandomycin eingesetzt. Ferner können zur Realisierung der Erfindung Substanzen eingesetzt werden, die die Expression von Genen hemmen, welche für Cytochrom P450 (CYP)-Monooxygenasen Isoformen, wie z.B. CYP2A6/7, CYP4A11, CYP2J2, oder P450 Aromatase, kodieren.

Als Steroid-Reduktasen Hemmer sind insbesondere 5-alpha-Steroid-Reduktase Hemmer, und hier vor allem Finasterid, geeignet.

Als Isomerasen Hemmer wird vorzugsweise Cyproteronacetat eingesetzt.

Als 17-beta-Hydroxy-Steroid-Dehydrogenasen Hemmer kommen bevorzugt 3beta-peptido-3alpha-hydroxy-5alpha-androstan-17-on-Derivate, Substanzen mit einer 17-spiro-gamma-lacton Gruppe, 3beta-[(N-adamantylmethyl-N-butanoyl)aminomethyl]-3 alpha-hydroxy-5-alpha-androstan-17-on, in Betracht.

Als 3-beta-Hydroxy-Steroid-Dehydrogenasen Hemmer werden vorzugsweise Cyproteroneacetate, als Aromatase Hemmer vorzugsweise Tamoxifen, Fadrozol, Aminoglutethimid, Formestan, Testolacton, 1,4,5-androsatriene-3,17-dione eingesetzt.

Das erfindungsgemäße Mittel kann weiterhin eine Substanz aus den nachfolgend genannten Gruppen, die die Folgeprodukte des Testosteronabbaus und deren Wirkung hemmen und/oder eliminieren enthalten:
- Substanzen, die an den Androgenrezeptor binden und eine Regulierung im Sinne einer Normalisierung der Rezeptordichte bewirken;
- Substanzen, die an den Androgenrezeptor binden und eine Regulierung im Sinne einer Hemmung der Androgenrezeptor vermittelten Genexpression, wie sie in der Herzhypertrophie beobachtet wird, bewirken, wobei vorzugsweise Antagonisten des Androgenrezeptors; und/oder Liganden des Androgenrezeptors eingesetzt werden.

Als Antagonisten des Androgenrezeptors werden vorzugsweise Flutamid, Bicalutamid eingesetzt und als Liganden des Androgenrezeptors vorzugsweise chirale und nichtchirale Bicalutamid Analoga, chirale und nichtchirale Bicalutamid Analoga mit einer Trifluormethyl-Gruppe, chirale und nichtchirale Bicalutamid Analoga mit einer Sulfid-Gruppe.

Weiterhin kann das erfindungsgemäße Mittel alpha-MHC und/oder Substanzen, die die Expression von alpha-MHC erhöhen, enthalten.

Nachfolgend wird die Erfindung anhand von Ausführungsbeispielen und Abbildungen näher erläutert.

### Beschreibung der Versuche

### Ausführungsbeispiel 1:

In Zellkultur-Experimenten mit isolierten Herzmuskelzellen der Ratte kann eine Reduktion und Normalisierung der Androgenrezeptorexpression nach Hinzugabe von Testosteron in das Kulturmedium erzielt werden (Abbildung 5c).

### Ausführungsbeispiel 2:

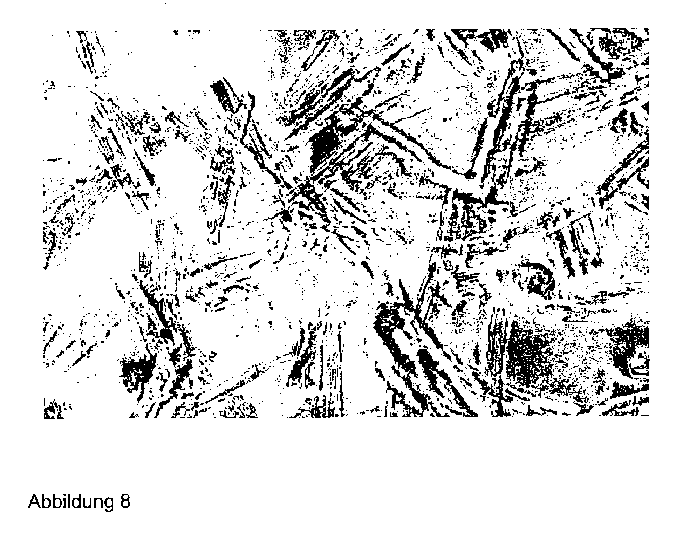

In Experimenten mit Herzmuskelzellen der Ratte wird der therapeutische Effekt einer Testosteronsubstitution, die zu einer Normalisierung der alpha-MHC-Expression führt, belegt (Abbildungen 7 und 8).

### Ausführungsbeispiel 3:

Eine etablierte Herzhypertrophie bildet sich nach Implantation bspw. eines linksventrikulären Unterstützungssystems (LVAD) zurück (Zafeiridis et al., 1998). Untersuchungen mit LVAD Implantatgewebe sind deshalb besonders wertvoll, weil die Patienten zunächst eine differentialdiagnostisch gesicherte Herzhypertrophie haben, die sich aber nach längerer Zeit aufgrund der Unterstützung durch das Assist Device zurückbildet und damit sich das Herz wieder normalisiert. Es wurde deshalb die Verstoffwechselung von Testosteron in LVAD unterstützen Herzen und konventionell behandelten Patienten untersucht. Im Vergleich zu gesunden und Patienten mit LVAD Implantaten war die Verstoffwechselung von Testosteron zu den verschiedenen Metaboliten (5-alpha-DHT, verschiedene Hydroxylierungsprodukte) in hypertrophem Herzgewebe deutlich erhöht (Abb. 3). Damit belegen wir, dass die Herzhypertrophie mit einem verändertem Steroidstoffwechsel des Testosterons in einem ursächlichen Zusammenhang steht.

### Ausführungsbeispiel 4:

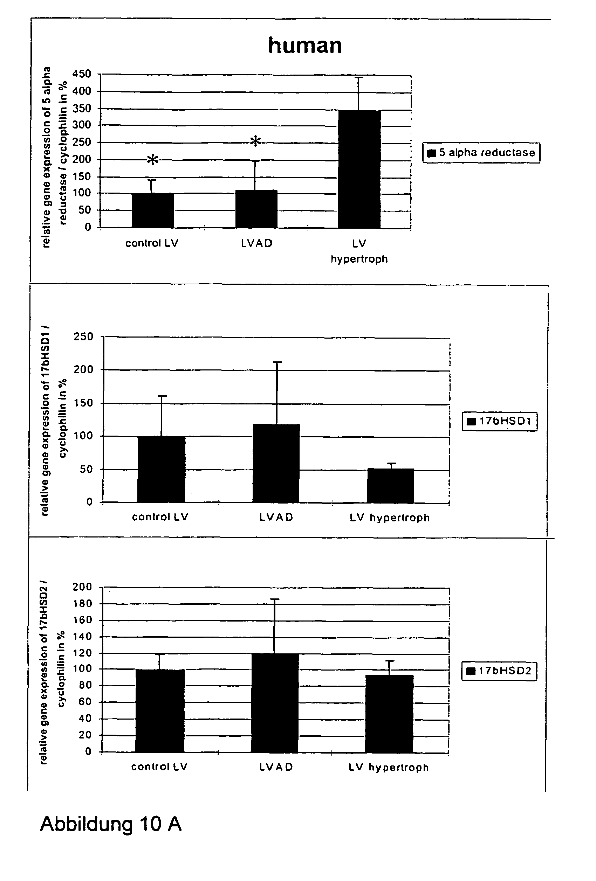

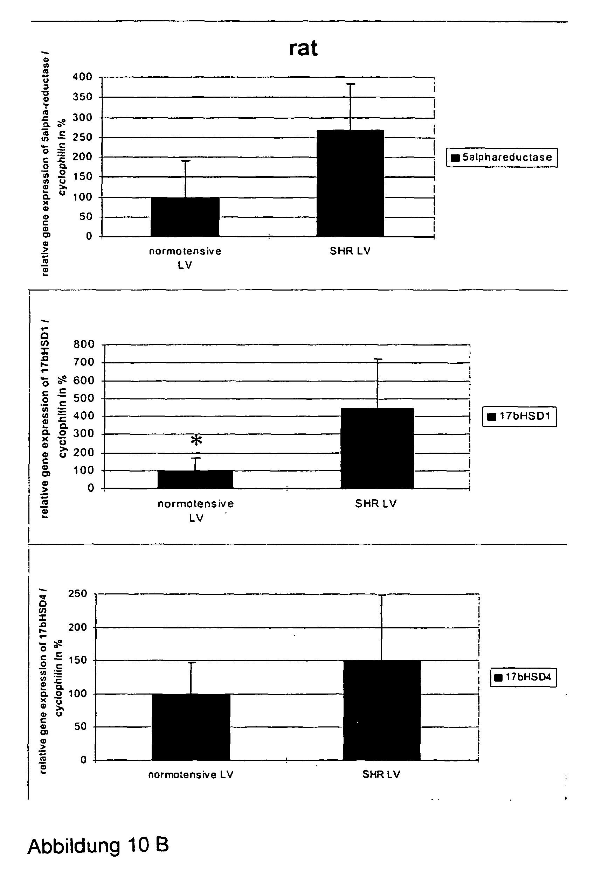

Es wird gezeigt, dass die geringere Metabolisierung von Testosteron in gesunden und LVAD behandeltem Herzgewebe mit einer verringerten Expression verschiedener Cytochrom P450 Monooxygenasen (CYP2A6/7, CYP2J2, CYP4A11) einhergeht (Abbildung 2).
Weiterhin sind die Gene c-jun, renin, 5-alpha-Reduktase und die Expression des Androgen Rezeptors in LVAD unterstützten Herzen im Vergleich zu hypertrophen Herzen stark reprimiert (Abbildungen 2 und 10), während alpha-MHC in gesunden und LVAD unterstütztem und damit druckentlastetem Herzgewebe verstärkt exprimiert wird (Abbildung 7). Damit ist der Beweis erbracht, dass die metabolischen Entgleisungen des Steroidstoffwechsels in der Herzhypertrophie nach LVAD Implantation normalisiert werden können und auch echokardiographisch ein Rückgang der Herzhypertrophie nach LVAD implantation klinisch belegt werden kann (Zafeiridis et al., 1998).

Zusammenfassend kann deshalb gesagt werden, dass der Steroidstoffwechsel in hypertrophen Herzen des Menschen, aber auch in entsprechenden Krankheitsmodellen der Ratte völlig unerwartet und überraschend stark verändert ist. Die zur Erfindung führenden Experimente dokumentieren eine krankheitsbedingte Induktion von CYP Monooxygenasen, sowie von weiteren am Testosteronstoffwechsel beteiligten Enzymen und belegen die klinische Relevanz der Untersuchungen anhand von Geweben und subzellulären Fraktionen hypertropher und LVAD implantierter Herzgewebsbiopsien. Untersuchungen mit isolierten Herzmuskelzellen belegen eindeutig den therapeutischen Effekt von Testosteron selbst, wie es am Beispiel des alpha-MHC kodierenden Genes gezeigt werden konnte. Die im klinischen Verlauf beobachtete Repression der alpha-MHC Genexpression ist mit der Herzhypertrophie gut korreliert und wird deshalb als biologischer Marker anerkannt (Nakao et al., 1997). Weitere Untersuchungen an explantierten Herzen und experimentellen Arbeiten im Rattenmodell belegen eine Fehlregulation des Androgenrezeptors in hypertrophen Herzen. Die in hypertrophierten Herzen beobachtete Induktion des Androgenrezeptors ist eine biologische Antwort / Adaptation auf die stark gesunkenen Testosteronspiegel des Herzgewebes. Testosteron und 5-alpha-Dihydrotestosteron aktivieren den Androgenrezeptor unterschiedlich und regulieren dadurch unterschiedliche Androgen-Rezeptor-responsive Gene. Somit werden durch die unterschiedlichen Liganden (Testosteron versus Dihydrotestosteron) des Rezeptors unterschiedliche Targetgene aktiviert. In der Herzhypertrophie ist weniger Rezeptor verfügbar, der an die responsiven Elemente des Promotors des alpha-MHC Gens bindet und damit wird alpha-MHC dereguliert. Eine geringe Expression von alpha-MHC führt aber zu einer negativen Inotropie und verschlechterten Herzleistung, wie sie auch im Falle der fortgeschrittenen Herzhypertrophie und der Herzinsuffizienz klinisch beobachtet wird.

**Tabelle 1A: Benutzte Oligonukleotide in Experimenten mit humanem Gewebe**

| **Genbank Zugangsnummer** | **Bezeichnung des Gens** | **Vorwärts Primer (5'-3')** | **Rückwärts Primer (5'-3')** | **Produktlänge (bp)** |
|---|---|---|---|---|
| | **Cytochrome P450 Gene** | | | |
| D01150 | CYP 1A1 | TCACAGACACAGCCTGATTGAG | GATGGGTTGACCCATAGCTT | 432 |
| M38504 | CYP 1A2 | TGGCTTCTACATCCCCAAGAAAT | TTCATGGTCAGCCCGTAGAT | 308 |
| U22027 | CYP 2A6/7 | GTGTGGACATGATGCCGT | AGGACTTGAGGCGGAAGT | 1151 |
| X13494 | CYP 2B6/7 | CCATACACAGAGGCAGTCAT | GGTGTCAGATCGATGTCTTC | 357 |
| XM_050922 | CYP 2C8 | GATCATGTAATTGGCAGACACA | CCTGCTGAGAAAGGCATGAAG | 311 |
| XM_050918 | CYP 2C9 | AGCTTGGAAAACACTGCAGT | CCTGCTGAGAAAGGCATGAAG | 437 |
| NM_000772 | CYP 2C18 | CTGTAACTGATATGTTTGGG | CCTGCTGAGAAAGGCATGAAG | 431 |
| NM_000769 | CYP 2C19 | GTAATCACTGCAGCTGACTTAC | CCTGCTGAGAAAGGCATGAAG | 431 |
| M33189 | CYP 2D6 | TGATGAGAACCTGCGCATAG | ACCGATGACAGGTTGGTGAT | 332 |
| AF084225 | CYP 2E1 | AGCACAACTCTGAGATATGG | ATAGTCACTGTACTTGAACT | 365 |
| J02906 | CYP 2F1 | ATGAACTTGCCGCACCGCGT | ACAGGCTCCACTTACGGTGC | 283 |
| X12387 | CYP 3A4 | CCAAGCTATGCTCTTCACCG | TCAGGCTCCACTTACGGTGC | 323 |
| L26985 | CYP 3A5 | TGTCCAGCAGAAACTGCAAA | TTGAAGAAGTCCTTGCGTGTC | 472 |
| NM 000765.1 | CYP 3A7 | CTATGATACTGTGCTACAGT | TCAGGCTCCACTTACGGTCT | 474 |
| AF208532 | CYP 4A11 | CAAGTGACCTCCCTGCTCAT | CTGATCTCCCCAGAATCACC | 280 |
| NM 000779.1 | CYP 4B1 | TGACCATGTGCATCAAAGGAG | AAAGCCATTCTTGGAGCGCA | 397 |
| | **17 beta hydroxy-steroid-dehydrogenase Gene** | | | |
| NM_000413 | 17b-HSD I | CAGCTGGACGTAAGGGACTC | AACTTGCTGGCGCAATAAAC | 293 |
| XM_012571 | 17b-HSD II | GAGCTTTCCAAGTGGGGAAT | AGCAAGGCAGATCCACAAGT | 299 |
| XM_005568 | 17b-HSD III | CCTCCGTAGTCAAGATGACA | CAAGGCAGCCACAGGTTTCA | 333 |
| XM_003904 | 17b-HSD IV | TATGCCCTGGCTTTTGCAGA | CAGTCTTCATCACTTATCCT | 275 |
| XM_001544 | **3 beta hydroxy-steroid-dehydrogenase I** | AGAGGCCTGTGTCCAAGCTA | GTTGTTCAGGGCCTCGTTTA | 298 |
| M32313 | **5 alpha reductase Marker genes for cardiac hypertrophy** | ATGTTCCTCGTCCACTACGG | GCCTCCCCTTGGTATTTTGT | 300 |
| AH002979 | renin | TTTGACACTGGTTCGTCCAA | CTGCCAGACACCAGTCTTGA | 498 |
| J04111 | c-jun | GAGCGGACCTTATGGCTACA | CCGTTGCTGGACTGGATTAT | 192 |
| XM_010429 | **Androgen receptor** | GTGGAAGCTGCAAGGTCTTC | GTTGTTGTCGTGTCCAGCAC | 350 |
| D00943 | **alpha MHC** | TACTGAGCAGCTAGGAGAAG | TCTGCCTTGATCTGGTTGAA | 177 |
| | **Housekeeping Gene** | | | |
| J02624 | GAPDH | GGCCAAGGTCATCCATGA | TCAGTGTAGCCCAGGATG | 353 |
| NM_021130 | cyclophilin | CTTGCCATTCCTGGACCCAA | TTTCGTGCTCTGAGCACTGG | 347 |

**Tabelle 1B: Benutzte Oligonukleotide in Experimenten mit Ratten-Gewebe**

| **Genbank Zugangsnummer** | **Bezeichnung des Gens** | **Vorwärts Primer (5'-3')** | **Rückwärts Primer (5'-3')** | **Produktlänge (bp)** |
|---|---|---|---|---|
| | **Cytochrome P450 Gene** | | | 331 |
| X00469 | CYP 1A1 | CTGGTTCTGGATACCCAGCTG | CCTAGGGTTGGTTACCAGG | 236 |
| X01031 | CYP 1A2 | GTCACCTCAGGGAATGCTGTG | GTTGACAATCTTCTCCTGAGG | 350 |
| NM_012940 | CYP 1B1 | CTCTCCCCCATCCACATCTA | AGACGAACATGACCCCTACG | 275 |
| NM_012692 | CYP 2A1/2 | CACAGGGCAGCTCTATGACA | CAGACCCAGCAAAGAAGAGG | 549 |
| L00316 | CYP 2B1/2 | GAGTTCTTCTCTGGGTTCCTG | ACTGTGGGTCATGGAGAGCTG | 248 |
| U33173 | CYP 2C11 | CTGCTGCTGCTGAAACACGTG | GGATGACAGCGATACTATCAC | 473 |
| M20131 | CYP 2E1 | CTCCTCGTCATATCCATCTG | GCAGCCAATCAGAAATGTGG | 364 |
| U39943 | CYP 2J3 | GGCATGCCCTTAATCAAAGA | AGCCTCAGCATCTCCTGAAA | 579 |
| X64401 | CYP 3A1 | ATCCGATATGGAGATCAC | GAAGAAGTCCTTGCTTGC | 116 |
| X79320 | CYP3A2 | CGACTTGGAACCCATAGAC | GGCTTAGGGAGATTTGACATG | 344 |
| M57718 | CYP 4A1 | GGTGACAAAGAACTACAGC | AGAGGAGTCTTGACCTGCCAG | |
| | **17 beta hydroxy-steroid-dehydrogenase Gene** | | | 299 |
| NM_012851 | 17b-HSD I | CCTCTTTGCCCACTATCAGC | GGAGACAAATGAGGGCTCTG | 295 |
| NM_024391 | 17b-HSD II | TGTGCTGGTGACAGGTG | ACGGTCATGGGAATGAGTTC | 333 |
| NM_054007 | 17b-HSD III | CCTCCGTAGTCAAGATGACA | CAAGGCAGCCACAGGTTTCA | 275 |
| NM_024392 | 17b-HSD IV | TATGCCCTGGCTTTTGCAGA | CAGTCTTCATCACTTATCCT | 292 |
| NM_012584 | **3 beta hydroxy-steroid-dehydrogenase I** | ATTCCAGTTGGAGCCTTCCT | CCCTCTTCCCATCATTGAGA | 308 |
| J05035 | **5 alpha reductase Marker genes for cardiac hypertrophy** | CCTTCCTGTTCTGCACCTTC | TGCAAACACTACACCCTGGA | 279 |
| NM_012642 | renin | CCGTGGTCCTCACCAACTAC | TTCCACCCACAGTTACCACA | 347 |
| X17163 | c-jun | CGACCAGAACGATGGACTTT | GGGTAGGACACCCAAACAAA | 381 |
| M23264 | **Androgen receptor** | GGTAATATCCGAAGGCAGCA | CAGCCCAGACTCTCACCTTC | 334 |
| X15938 | **alpha MHC** | GGAAGAGCGAGCGGCGCATCAAGG | CTGCTGGACAGGTTATTCCTCA | 353 |
| | **Housekeeping Gene** | | | |
| X02231 | **GAPDH** | GGCCAAGGTCATCCATGA | TCAGTGTAGCCCAGGATG | 347 |
| M19533 | cyclophilin | CTTGCCATTCCTGGACCCAA | TTTCGTGCTCTGAGCACTGG | |

### Referenzen

[No authors listed] Risks and benefits of estrogen plus progestin in healthy postmenopausal women: principal results From the Women's Health Initiative randomized controlled trial. JAMA. 2002 Jul 17;288(3):321-33.
Arver S, Dobs AS, Meikle AW, Caramelli KE, Rajaram L, Sanders SW, Mazer NA. Long-term efficacy and safety of a permeation-enhanced testosterone transdermal system in hypogonadal men. Clin Endocrinol (Oxf). 1997 Dec;47(6):727-37.
Arlotto MP, Trant JM, Estabrook RW. Measurement of steroid hydroxylation reactions by high-performance liquid chromatography as indicator of P450 identity and function. Methods Enzymol. 1991;206:454-62.
Brawley OW, Barnes S, Parnes H. The future of prostate cancer prevention. Ann N Y Acad Sci. 2001 Dec;952:145-52.
Cai LQ, Fratianni CM, Gautier T, Imperato-McGinley J. Dihydrotestosterone regulation of semen in male pseudohermaphrodites with 5 alpha-reductase-2 deficiency. J Clin Endocrinol Metab. 1994 Aug;79(2):409-14.
Claessens F, Verrijdt G, Schoenmakers E, Haelens A, Peeters B, Verhoeven G, Rombauts W. Selective DNA binding by the androgen receptor as a mechanism for hormone-specific gene regulation. J Steroid Biochem Mol Biol. 2001 Jan-Mar;76(1-5):23-30.
Cleutjens KB, van Eekelen CC, van der Korput HA, Brinkmann AO, Trapman J. Two androgen response regions cooperate in steroid hormone regulated activity of the prostate-specific antigen promoter. J Biol Chem. 1996 Mar 15;271(11):6379-88.
Eicheler W, Happle R, Hoffmann R. 5 alpha-reductase activity in the human hair follicle concentrates in the dermal papilla. Arch Dermatol Res. 1998 Mar;290(3):126-32.
English KM, Steeds RP, Jones TH, Diver MJ, Channer KS. Low-dose transdermal testosterone therapy improves angina threshold in men with chronic stable angina: A randomized, double-blind, placebo-controlled study. Circulation. 2000 Oct 17;102(16):1906-11.
Howell S, Shalet S. Testosterone deficiency and replacement. Horm Res. 2001;56 Suppl 1:86-92.
Karvonen U, Kallio PJ, Janne OA, Palvimo JJ. Interaction of androgen receptors with androgen response element in intact cells. Roles of amino- and carboxylterminal regions and the ligand. J Biol Chem. 1997 Jun 20;272(25):15973-9.
Krause W. [Teratogenic damages of the male genital organs] Fortschr Med. 1976 Oct 21;94(30):1673-5.
Ly LP, Handelsman DJ. Muscle strength and ageing: methodological aspects of isokinetic dynamometry and androgen administration. Clin Exp Pharmacol Physiol. 2002 Jan-Feb;29(1-2):37-47.
Mason RA, Morris HA. Effects of dihydrotestosterone on bone biochemical markers in sham and oophorectomized rats. J Bone Miner Res. 1997 Sep;12(9):1431-7.
Miyata K, Yabushita S, Sukata T, Sano M, Yoshino H, Nakanishi T, Okuno Y, Matsuo M. Effects of perinatal exposure to flutamide on sex hormones and androgen-dependent organs in F1 male rats. J Toxicol Sci. 2002 Feb;27(1):19-33.
O'Donnell L, Pratis K, Stanton PG, Robertson DM, McLachlan RI. Testosteronedependent restoration of spermatogenesis in adult rats is impaired by a 5alpha-reductase inhibitor. J Androl. 1999 Jan-Feb;20(1):109-17.
Nakao K, Minobe W, Roden R, Bristow MR, Leinwand LA. Myosin heavy chain gene expression in human heart failure. J Clin Invest. 1997 Nov 1;100(9):2362-70.
Parker TG, Chow KL, Schwartz RJ, Schneider MD. Differential regulation of skeletal alpha-actin transcription in cardiac muscle by two fibroblast growth factors. Proc Natl Acad Sci U S A. 1990 Sep;87(18):7066-70.
Porto CS, Lazari MF, Abreu LC, Bardin CW, Gunsalus GL. Receptors for androgen-binding proteins: internalization and intracellular signalling. J Steroid Biochem Mol Biol. 1995 Jun;53(1-6):561-5.
Poujol N, Wurtz JM, Tahiri B, Lumbroso S, Nicolas JC, Moras D, Sultan C. Specific recognition of androgens by their nuclear receptor. A structure-function study. J Biol Chem. 2000 Aug 4;275(31):24022-31.
Roberts AB, Roche NS, Winokur TS, Burmester JK, Sporn MB. Role of transforming growth factor-beta in maintenance of function of cultured neonatal cardiac myocytes. Autocrine action and reversal of damaging effects of interleukin-1. J Clin Invest. 1992 Nov;90(5):2056-62.
Rosenfield RL, Deplewski D, Kentsis A, Ciletti N. Mechanisms of androgen induction of sebocyte differentiation. Dermatology. 1998;196(1):43-6.
Rosano GM, Panina G. Oestrogens and the heart. Therapie. 1999 May-Jun;54(3):381-5.
Sairam MR, Krishnamurthy H. The role of follicle-stimulating hormone in spermatogenesis: lessons from knockout animal models. Arch Med Res. 2001 Nov-Dec;32(6):601-8.
Schroder FH. 5 alpha-reductase inhibitors and prostatic disease. Clin Endocrinol (Oxf). 1994 Aug;41 (2):139-47.
Schulman CC. Long-term aspects of medical treatment of BPH. Eur Urol. 2001;40 Suppl 3:8-12.
Sinha-Hikim I, Artaza J, Woodhouse L, Gonzalez-Cadavid N, Singh AB, Lee MI, Storer TW, Casaburi R, Shen R, Bhasin S. Testosterone-induced increase in muscle size in healthy young men is associated with muscle fiber hypertrophy. Am J Physiol Endocrinol Metab. 2002 Jul;283(1):E154-64.
Skelton, R., Gill, A.B. and Parsons, J.M. (1998) Cardiac effects of short course dexamethasone in preterm infants. Arch Dis Child Fetal Neonatal Ed 78, 133-7.
Smith PK, Krohn RI, Hermanson GT, Mallia AK, Gartner FH, Provenzano MD, Fujimoto EK, Goeke NM, Olson BJ, Klenk DC. Measurement of protein using bicinchoninic acid. Anal Biochem. 1985 Oct;150(1):76-85.
Sugden PH, Bogoyevitch MA. Intracellular signalling through protein kinases in the heart. Cardiovasc Res. 1995 Oct;30(4):478-92.
Thum T, Borlak J. Cytochrome P450 mono-oxygenase gene expression and protein activity in cultures of adult cardiomyocytes of the rat. Br J Pharmacol. 2000a Aug;130(8):1745-52.
Thum T, Borlak J. Gene expression in distinct regions of the heart. Lancet. 2000b Mar 18;355(9208):979-83.
Whiting DA. Advances in the treatment of male androgenetic alopecia: a brief review of finasteride studies. Eur J Dermatol. 2001 Jul-Aug;11(4):332-4.
Zafeiridis A, Jeevanandam V, Houser SR, Margulies KB. Regression of cellular hypertrophy after left ventricular assist device support. Circulation. 1998 Aug 18;98(7):656-62.

### Legenden zu den Abbildungen

Abb. 1) Schematische Darstellung der deregulierten Testosteron-Verstoffwechselung im hypertrophen humanen Herzen
Abb. 2A -2G) Semiquantitative Echtzeit RT-PCR Untersuchungen zur herzspezifischen Expression verschiedener Gene in humanen gesunden, hypertrophierten und Herzen mit linksventrikulären Unterstützungssystemen (LVAD). Die genauen Details sind der "Beschreibung der Versuche, Allgemeines", sowie der Tabelle 1A zu entnehmen.
Abb. 3A und 3B) Metabolismus von Testosteron in mikrosomalen Suspensionen humaner Herzen und von Rattenherzen. Die Zugabe des 5-alpha-Steroidreduktase Hemmers Finasteride (25µM) führte zu einer 60%igen Reduktion der 5-alpha-DHT Bildung.
   Die Isolation mikrosomaler Membranen aus ventrikulärem Gewebe wurde wie folgt durchgeführt.
   Human- und Rattenherzgewebe (Ventrikel) wurde in kleine (ca. 1mm³) Stücke zerschnitten und mittels eines Ultraturrax (IKA, Deutschland) in KCl-Puffer (0,15M, pH7,4) bei 4°C homogenisiert. Die weitere Isolation erfolgte wie in Thum und Borlak (2000b) beschrieben. Die mikrosomale Suspension wurde in einen TRIS-Sucrose Puffer (0,25M Sucrose, 20mM TRIS-Puffer, 5mM EDTA) überführt und bis zur weiteren Verwendung bei -80°C gelagert.
   Dir Proteinkonzentration in den isolierten Mikrosomen des Herzens wurde nach der Methode von Smith et al. (1985) bestimmt und auf 1mg/ml eingestellt.
   500µg mikrosomales Protein wurde mit 100µM Testosteron und 1mg/ml NADPH in einem Gesamtvolumen von 1ml TRIS-Puffer (20mM, Sigma, Deisenhofen, Deutschland) für 4h bei 37°C in einem Schüttelwasserbad inkubiert und anschliessend in Flüssigstickstoff schockgefroren und bis zur weiteren Analyse bei -80°C gelagert. Zu einigen Proben wurde vor Inkubation 25µM Finasteride hinzugegeben.
Abb. 4A und 4B) HPLC (Hochdruckflüssigkeitschromatographie) Chromatogramme nach Verstoffwechselung von Testosteron (100µM) in mikrosomalen Suspensionen hypertropher Herzen und Herzen mit linksventrikulären Unterstützungssystemen (LVAD).
   Testosteron und seine Metabolite wurden mittels HPLC nach einer leicht veränderten Methode von Arlotto et al. (1991) analysiert. Vor Analyse der Proben wurde als interner Standard 11-alpha-Hydroxyprogesteron (1µg) hinzugegeben. Nach Zugabe von 100µl Isopropanol zu 1 ml der zu analysierenden Probe, wurde diese durch Ausschütteln (20min) mit 5ml Ethylacetat extrahiert. Die Extrakte wurden durch Stickstoffbeblasung eingetrocknet und dann mit 100µl der mobilen HPLC-Phase (H₂O/Methanol/Acetonitril, 60/25/15, v/ v/ v) aufgenommen. Anschließend wurden 80µl der Probe in das HPLC System (Hewlett Packard HP1100) injiziert. Die Durchlaufgeschwindigkeit betrug 1ml/min. Zur chromatographischen Auftrennung der Metabolite wurde eine C18 Nucleosil Cromatographiesäule eingesetzt (250 x 4mm, Partikelgröße 5µm; Macherey-Nagel, Deutschland). Die Testosteronmetabolite wurden bei einer Temperatur von 30°C mittels UV Absorptionsdetektion bei Wellenlängen von 246nm und 285nm (zum Nachweis von Dihydrotestosteron) unter Zuhilfenahme synthetischer Referenzsubstanzen analysiert und quantifiziert.
   Die mobile Phase bestand aus H₂O (A), Methanol (B) und Acetonitril (C) und die HPLC Messung wurde zuerst für 12min mit einer isokratischen Elution aus 60% A, 25% B und 15% C, anschließend mit 45% A, 40% B und 15% C für 3min und schließlich mit 45% A, 45% B und 10% C. Die gesamte Laufzeit betrug 45 min pro Probe.
Abb. 5A, 5B und 5C) Semiquantitative Echtzeit RT-PCR Untersuchungen zur herzspezifischen Expression des Androgen Rezeptors in humanen gesunden, hypertrophierten und Herzen mit linksventrikulären Unterstützungssystemen (A) und in nicht hypertrophierten und hypertrophen Herzen der Ratte (B). C zeigt die Androgenrezeptor Expression vor und nach Testosteron Behandlung (100µM) von kultivierten Herzmuskelzellen der Ratte. Die genauen Details sind der "Beschreibung der Versuche, Allgemeines", sowie den Tabellen 1A und 1B zu entnehmen.

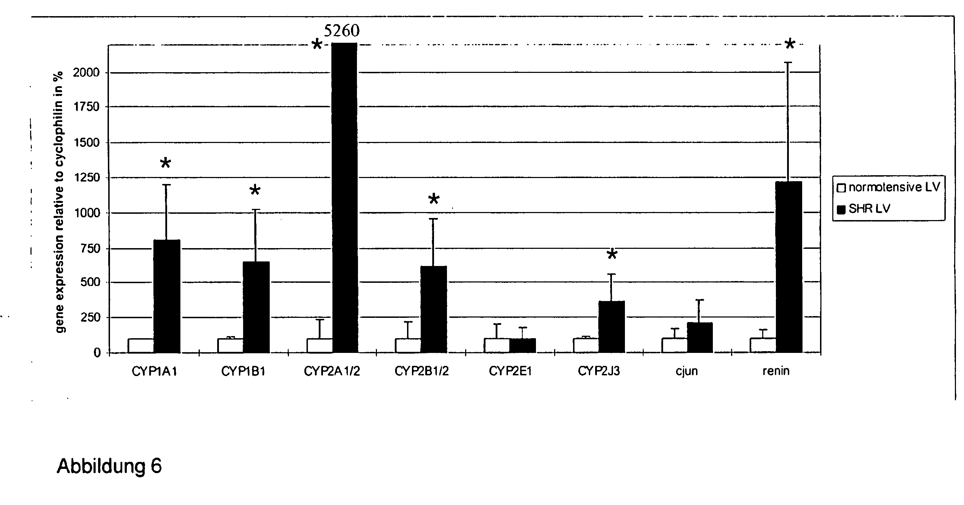

Abb. 6) Semiquantitative Echtzeit RT-PCR Untersuchungen zur herzspezifischen Expression verschiedener Gene in gesunden und hypertrophierten Herzen der Ratte (SHR). Die genauen Details sind der "Beschreibung der Versuche, Allgemeines", sowie den Tabellen 1A und 1B zu entnehmen.
Abb. 7A, 7B und 7C) Semiquantitative Echtzeit RT-PCR Untersuchungen zur herzspezifischen Expression des alpha-MHCs in humanen gesunden, hypertrophierten Herzen und Herzen mit linksventrikulären Unterstützungssystemen (A) und in nicht hypertrophierten und hypertrophen Herzen der Ratte (B). C zeigt die alpha-MHC Expression vor und nach Testosteron Behandlung (100µM) von kultivierten Herzmuskelzellen der Ratte. Die Isolation von Herzmuskelzellen adulter Ratten wurde durchgeführt, wie von Thum und Borlak (2000a) beschrieben. 24h nach Isolation wurden die kultivierten Herzzellen mit 100µM Testosteron für 8h behandelt, anschließend entfernt und 5min mit 1200RPM und 4°C zentrifugiert. Das resultierende Zellpellet wurde in flüssigem Stickstoff schockgefroren und bis zur weiteren Analyse bei -80°C gelagert.
   Die genauen Details der RT-PCR Untersuchungen sind der "Beschreibung der Versuche, Allgemeines", sowie den Tabellen 1A und 1B zu entnehmen.
Abb. 8) Phasenkontrastmikroskopische Aufnahme von frisch isolierten Herzmuskelzellen der Ratte.
Abb. 9) Potentielle Androgenrezeptor Bindungsstellen im Promotor des alpha-MHC Gens des Menschen, der Ratte und der Maus.
   Mittels der GEMS Launcher Release 3.0 Software (Genomatix, Deutschland) wurde eine Matrix für die Androgenrezeptor Bindungsstelle konstruiert. Dafür wurden Sequenzinformationen aus der Transkriptionsfaktoren Datenbank TRANSFAC 4.0 (http://transfac.gbf.de/TRANSFAC/) und aus verschiedenen Publikationen (Karvonen et al., 1997, Claessens et al., 2001; Cleutjens et al., 1996) benutzt und so konnte eine Matrix für die Androgenrezeptor-Bindungsstelle mit hochkonservierten Basen (5'-XGXXCGGGGAGTTCT-3') entwickeln werden. Mit dieser Matrix konnten im humanen, Ratten- und Maus-Promotor des alpha-MHC Genes Bindungsstellen für den Androgen-Rezeptor identifiziert werden.
Abb. 10A und 10B) Semiquantitative Echtzeit RT-PCR Untersuchungen zur herzspezifischen Expression verschiedener Gene in humanen (Abb. 10A) gesunden, hypertrophierten und Herzen mit linksventrikulären Unterstützungssystemen (LVAD) und in nicht hypertrophierten und hypertrophen Herzen der Ratte (Abb. 10B). Die genauen Details der RT-PCR Untersuchungen sind der "Beschreibung der Versuche, Allgemeines", sowie den Tabellen 1A und 1B zu entnehmen.

## Patentansprüche

1. Verwendung eines Mittels für die Herstellung eines Arzneimittels zur Therapie von Herzhypertrophie, **dadurch gekennzeichnet, dass** das Mittel mindestens eine Substanz enthält, die die Testosteron-Konzentration in den erkrankten Geweben auf den Normalwert erhöhen, wobei die Substanz eine der Substanzen
- Testosteron,
- Testosteron in Form von Testosteron-Estern, 19-Nortestosteron, Testosteronpropionat, Testosteronundecanoat, Mesterolon, Fluoxymesteron,
- Nandrolondecanoat, Clostebolacetat, Metenololonacetat, Androstendion, Dehydroepiandrosteron,
- Cimetidin, Fluoroquinolone, Fluvoxamin, Furafyllin, Methoxsalen, Mibefradil, Ticlopidin, Thiotepa, Felbamat, Fluoxetin, Indomethacin, Ketoconazol, Lansoprazol, Modafinil, Omeprazole, Paroxetin, Probenicid, Topiramat, Fluconazol, Fluvastatin, Isoniazid, Phenylbutazon, Sertralin, Sulfamethoxazol, Sulfaphenazol, Teniposid, Trimethoprim, Zafirlukast, Clecoxib, Chlorpromazin, Chlorpheniramin, Clomipramin, Kocain, Doxorubicin, Halofantrin, Red-Haloperidol, Levomepromazin, Metoclopramid, Methadon, Mibefradil, Moclobemid, Ranitidin, Ritonavir, Terbinafin, Diethyl- Dithiocarbamat, Disulfiram, Delaviridin, Indinavir, Nelfinavir, Saquinavir, Ciprofloxacin, Clarithromycin, Erythromycin, Gestoden, Grapefruit Saft, Itraconazol, Mifepriston, Nefazodon, Norfloxacin, Norfluoxetin, Troleandomycin,
- Finasterid,
- Cyproteronacetat,
- 3beta-peptido-3alpha-hydroxy-5alpha-androstan-17-on-Derivate, Substanzen mit einer 17-spiro-gamma-lacton Gruppe, 3beta-[(N-adamantylmethyl-N-butanoyl)aminomethyl]-3 alpha-hydroxy-5-alpha-androstan-17-on,
- Tamoxifen, Fadrozol, Aminoglutethimid, Formestan,Testolacton oder 1,4,5-androsatriene-3,17-dione
ist.

## Claims

1. Use of an agent in the manufacture of a medicament for treating cardiac hypertrophy, **characterized in that** the agent contains at least one substance which increases the testosterone concentration in the diseased tissues to the normal level, wherein the substance is one of the substances
- testosterone,
- testosterone in the form of testosterone esters, 19-nortestosterone, testosterone propionate, testosterone undecanoate, mesterolone, fluoxymesterone,
- nandrolone decanoate, clostebol acetate, metenolone acetate, androstenedione, dehydroepiandrosterone,
- cimetidine, fluoroquinolones, fluvoxamine, furafylline, methoxsalen, mibefradil, ticlopidine, thiotepa, felbamate, fluoxetine, indomethacin, ketoconazole, lansoprazole, modafinil, omeprazols, paroxetine, probenecid, topiramate, fluconazole, fluvastatin, isoniazid, phenylbutazone, sertraline, sulfamethoxazole, sulfaphenazole, teniposide, trimethoprim, zafirlukast, celecoxib, chlorpromazine, chlorpheniramine, clomipramine, cocaine, doxorubicin, halofantrine, red-haloperidol, levomepromazine, metoclopramide, methadone, mibefradil, moclobemide, ranitidine, ritonavir, terbinafine, diethyldithiocarbamate, disulfiram, delavirdine, indinavir, nelfinavir, saquinavir, ciprofloxacin, clarithromycin, erythromycin, gestodene, grapefruit juice, itraconazole, mifepristone, nefazodone, norfloxacin, norfluoxetine, troleandomycin,
- finasteride,
- cyproterone acetate,
- 3beta-peptide-3alpha-hydroxy-5alpha-androstane-17-one derivatives, substances having a 17-spiro-gamma-lactone group, 3-beta-[N-adamantyl-methyl-N-butanoyl)aminomethyl]-3 alpha-hydroxy-5-alpha-androstane-17-one,
- tamoxifen, fadrozole, aminoglutethimide, formestane, testolactone, or 1,4,5-androstadiene-3,17-dione.

## Revendications

1. Utilisation d'un agent pour la production d'un médicament pour la thérapie de l'hypertrophie cardiaque, **caractérisée en ce que** le dit agent contient au moins une substance qui augmente la concentration de la testostérone dans les tissus pathologiques jusqu'au niveau physiologique, la dite substance étant une des substances
- testostérone,
- testostérone sous forme d'esters de testostérone, 19-nortestostérone, propionate de testostérone, undécanoate de testostérone, mestérolone, fluoxymestérone,
- décanoate de nandrolone, clostébol acétate, acétate de méténolone, androsténedione, déhydroépiandrostérone,
- cimétidine, des fluoroquinolones, fluvoxamine, furafylline, methoxsalène, mibéfradil, ticlopidine, thiotépa, felbamate, fluoxétine, indométhacine, kétoconazole, lansoprazol, modafinil, des oméprazoles, paroxétine, probénécide, topiramate, fluconazole, fluvastatine, isoniazide, phénylbutazone, sertraline, sulfaméthoxazole, sulfaphénazol, téniposide, triméthoprime, zafirlukast, célécoxib, chlorpromazine, chlorphéniramine, clomipramine, cocaïne, doxorubicine, halofantrine, halopéridol (à action retardée), lévomépromazine, métoclopramide, méthadone, mibéfradil, moclobémide, ranitidine, ritonavir, terbinafine, diéthyldithiocarbamate, disulfiram, délavirdine, indinavir, nelfinavir, saquinavir, ciprofloxacine, clarithromycine, érythromycine, gestodène, jus de pamplemousse, itraconazol, mifépriston, néfazodon, norfloxacine, norfluoxétine, troléandomycine,
- finastéride,
- acétate de cyprotérone,
- dérivés de 3-bêta-peptide-3-alpha-hydroxy-5-alpha-androstan-17-one, des substances ayant un groupe 17-spiro-gamma-lactone, 3-bêta-[N-adamantyl-methyl-N-butanoyl)aminomethyl]-3-alpha-hydroxy-5-alpha-androstan-17-one,
- tamoxifène, fadrozole, aminoglutéthimide, formestan, testolactone ou 1,4,5-androstadiènre-3,17-dione.
